(19) Europäisches Patentamt
European Patent Office
Office européen des brevets

(11) EP 0 644 943 B1

(12) **EUROPEAN PATENT SPECIFICATION**

(45) Date of publication and mention of the grant of the patent:
**05.10.2005 Bulletin 2005/40**

(21) Application number: **93912959.9**

(22) Date of filing: **11.06.1993**

(51) Int Cl.[7]: **C12N 15/82**, C12N 15/29, C12N 15/55, C12N 15/31, C12N 5/10, A01H 1/02

(86) International application number:
**PCT/EP1993/001489**

(87) International publication number:
**WO 1993/025695 (23.12.1993 Gazette 1993/30)**

# (54) MAINTENANCE OF MALE-STERILE PLANTS

ERHALTUNG VON MÄNNLICHEN STERILEN PFLANZEN

ENTRETIEN DE PLANTES STERILES MALES

(84) Designated Contracting States:
**AT BE CH DE DK ES FR GB GR IE IT LI LU MC NL PT SE**

(30) Priority: **12.06.1992 US 899072**
**03.11.1992 US 970840**

(43) Date of publication of application:
**29.03.1995 Bulletin 1995/13**

(73) Proprietor: **Bayer BioScience N.V.**
**9052 Gent (BE)**

(72) Inventors:
- **WILLIAMS, Mark**
  **B-9000 Gent (BE)**
- **LEEMANS, Jan**
  **B-9000 Gent (BE)**

(74) Representative: **Gutmann, Ernest et al**
**Ernest Gutmann - Yves Plasseraud S.A.**
**3, rue Chauveau-Lagarde**
**75008 Paris (FR)**

(56) References cited:
**EP-A- 0 412 911** **WO-A-90/08828**

- **NATURE vol. 357, 4 June 1992, LONDON GB pages 384 - 387 MARIANI, C., ET AL. 'A chimaeric ribonuclease-inhibitor gene restores fertility to male sterile plants'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 644 943 B1

Printed by Jouve, 75001 PARIS (FR)

## Description

This invention relates to a process for maintaining male-sterile plant lines that can be used for the production of hybrid seed of a crop, to maintainer plants that can be used in such a process, and to maintainer genes that can be used to produce such maintainer plants.

## Backaround of the Invention

In many, if not most, plant species, the development of hybrid cultivars is highly desired because of their generally increased productivity due to heterosis: the superior performance of hybrid individuals compared with their parents (see, e.g., Fehr (1987) "Principles of Cultivar Development, Volume 1: Theory and Technique", MacMillan Publishing Company, New York; Allard (1960) "Principles of Plant Breeding", John Wiley and Sons, Inc., New York).

The development of hybrid cultivars of various plant species depends upon the capability to achieve almost complete cross-pollination between parents. This is most simply achieved by rendering one of the parent lines male-sterile (i.e., with pollen being absent or nonfunctional), for example, by manually removing the one parent's anthers or by providing the one parent with naturally occurring cytoplasmic or nuclear genes that prevent anther and/or pollen development and/or function, using classical breeding techniques (for a review of the genetics of male-sterility in plants, see Kaul (1988) "Male Sterility in Higher Plants", Springer Verlag, New York).

For hybrid plants where the seed is the harvested product (e.g., corn and oilseed rape), it is, in most cases, also necessary to ensure that fertility of the hybrid plants is fully restored. In plants in which the male-sterility is under genetic control, this requires the use of genes that can restore male-fertility. Hence, the development of hybrid cultivars is mainly dependent on the availability of suitable and effective sterility and restorer genes.

Endogenous nuclear loci are known for most plant species that contain genotypes which effect male-sterility, and generally, such loci need to be homozygous for particular recessive alleles in order to result in a male-sterile phenotype. The presence of a dominant male-fertile allele at such loci results in male-fertility.

Recently, it has been shown that male-sterility can be induced in a plant by providing the plant with a nuclear male-sterility genotype that includes a chimaeric male-sterility gene comprising a DNA sequence (or male-sterility DNA) coding, for example, for a cytotoxic product (such as an RNase) and under the control of a promoter which is predominantly active in selected tissue of the plant's male reproductive organs. In this regard, tapetum-specific promoters, such as the promoter of the TA29 gene of Nicotiana tabacum, have been shown to be particularly useful for this purpose (Mariani et al (1990) Nature 347:737; European patent publication ("EP") 0,344,029). By providing the nuclear genome of the plant with such a male-sterility gene, an artificial nuclear male-sterility locus is created containing the artifical male-sterility genotype that results in a male-sterile plant.

In addition, it has been recently shown that male-fertility can be restored to such a nuclear male-sterile plant with a chimaeric fertility-restorer gene comprising another DNA sequence (or fertility-restorer DNA) that codes, for example, for a protein that inhibits the activity of the cytotoxic product or otherwise prevents the cytotoxic product from being active at least in the selected tissue of the plant's male reproductive organs (EP 0,412,911). For example, the barnase gene of Bacillus amyloliquefaciens codes for an RNase (Barnase) which can be inhibited by a protein (Barstar) that is encoded by the barstar gene of B. amyloliquefaciens. Hence, the barnase gene can be used for the construction of a chimaeric male-sterility gene while the barstar gene can be used for the construction of a chimaeric fertility-restorer gene. Experiments in different plant species (e.g., oilseed rape) have shown that such a chimaeric barstar gene can fully restore the male-fertility of male-sterile lines in which the male-sterility was due to the presence of such a chimaeric barnase gene (EP 0,412,911: Mariani et al (1991) Proceedings of the CCIRC Rapeseed congress, July 9-11, 1991 Saskatoon, Saskatchewan, Canada; Mariani et al (1992) Nature 357:384). By coupling a marker gene, such as a dominant herbicide resistance gene (for example, the bar gene coding for phosphinothricin acetyl transferase (PAT) that converts herbicidal phosphinothricin to a non-toxic compound [De Block et al (1987) EMBO J. 6:2513]), to the chimaeric male-sterility and/or fertility restorer gene, breeding systems can be implemented to select for uniform populations of male-sterile plants (EP 0,344,029; EP 0,412,911).

The production of hybrid seed of any particular cultivar of a plant species requires the: 1) maintenance of small quantities of pure seed of each inbred parent and 2) the preparation of larger quantities of seed of each inbred parent. Such larger quantities of seed would normally be obtained by several (usually two) seed-multiplication rounds, starting from a small quantity of pure seed ("basic seed") and leading, in each multiplication round, to a larger quantity of seed of the inbred parent and finally to a stock of seed of the inbred parent ("parent seed" or "foundation seed") which is of sufficient quantity to be planted to produce the desired quantities of hybrid seed. Of course, in each seed-multiplication round, larger planting areas (fields) are required.

In order to maintain and enlarge a small stock of seeds of male-sterile plants, it has been necessary to cross the parent male-sterile plants with normal pollen-producing parent plants. The offspring of such a cross will, in all cases, be a mixture of male-sterile and male-fertile plants, and the latter have to be removed from the former. With male-sterile plants containing an artificial male-sterility locus as described above, such removal can be facilitated by genetically linking the chimaeric male-sterility gene to a suitable marker gene, such as the bar gene, which allows the easy identification and removal of the male-fertile plants. EP 0,198,288 and US Patent 4,717,219, by comparison, describe methods for linking such marker genes (which can be visible markers or dominant conditional markers) to endogenous nuclear loci containing male-sterility genotypes.

However, even when suitable marker genes are linked to male-sterility genotypes, the maintenance of parent male-sterile plants still requires the removal from the field of a substantial number of plants. For instance, in systems using a herbicide resistance gene (e.g., the bar gene) linked to a chimaeric male-sterility gene, only half of the parent stock will result in male-sterile plants, thus requiring the removal of the male-fertile plants by herbicide spraying prior to flowering. In any given field, the removal of male-fertile plants effectively reduces the potential yield of hybrid seed or the potential yield of male-sterile plants during each round of seed multiplication for producing of parent seed. This is economically unattractive for many important crop species such as corn and oilseed rape. In order to minimize the number of male-fertile plants which have to be removed, male-fertile maintainer plants have been sought which, when crossed with a male-sterile parent plant, produce a minimum, preferably no, male-fertile offspring, thereby minimizing or avoiding altogether the need to remove such male-fertile offspring. To solve an analogous problem, US Patents 3,710,511 and 3,861,079 have described procedures for producing and maintaining a homogenous population of male-sterile plants by using specific chromosomal abnormalities that are differentially transmitted to the egg and the sperm in the plants.

## Summary of the Invention

In accordance with this invention, a cell of a transgenic plant ("the maintainer plant") is provided, in which the nuclear genome contains stably integrated therein: 1) at a first locus or male-sterility locus, a male-sterility genotype in homozygous condition; and 2) at a second locus or maintainer locus, a maintainer gene in heterozygous condition; the male-sterility locus and the maintainer locus preferably being unlinked; the maintainer gene being a foreign DNA sequence, preferably a foreign chimaeric DNA sequence, containing:

a) a fertility-restorer gene that comprises at least:

i) a fertility-restorer DNA encoding a restorer RNA and/or protein or polypeptide which, when produced or overproduced in some or all of the cells, preferably stamen cells, of the plant, prevents phenotypic expression of the nuclear male-sterility genotype that would render the plant male-sterile in the absence of expression of the fertility-restorer DNA in the some or all stamen cells and
ii) a restorer promoter capable of directing expression of the fertility-restorer DNA at least in the some or all of the cells, preferably stamen cells, of the plant, so that the phenotypic expression of the nuclear male-sterility genotype is prevented, the fertility-restorer DNA being in the same transcriptional unit as, and under the control of, the restorer promoter and

b) a pollen-lethality gene that is selectively expressed in microspores and/or pollen of the plant to produce nonfunctional pollen and that comprises at least:

iii) a pollen-lethality DNA coding for a pollen-lethality RNA and/or protein or polypeptide that, when produced or overproduced in the microspores and/or pollen, significantly disrupts their metabolism, functioning and/or development and
iv) a pollen-specific promoter capable of directing expression of the pollen-lethality DNA selectively in the microspores and/or pollen of the plant, the pollen-lethality DNA being in the same transcriptional unit as, and under the control of, the pollen promoter.

The cell of the maintainer plant of this invention preferably also comprises, especially in the maintainer locus, at least one first marker gene which comprises at least:

v) a first marker DNA encoding a first marker RNA and/or protein or polypeptide which, when present at least in a first specific tissue or specific cells of the plant, renders the plant easily separable from other plants which do not contain the first marker RNA, protein or polypeptide encoded by the first marker DNA at least in the first specific tissue or specific cells and
vi) a first marker promoter capable of directing expression of the first marker DNA at least in the first specific tissue or specific cells, the first marker DNA being in the same transcriptional unit as, and under the control of, the first marker promoter.

The male-sterility genotype in the cell of the maintainer plant of this invention can be foreign or endogenous but is preferably a foreign, especially chimaeric, male-sterility gene which comprises:

1) a male-sterility DNA encoding a sterility RNA and/or protein or polypeptide which, when produced or overproduced in a stamen cell of the plant in the absence of the restorer RNA, protein or polypeptide, significantly disturbs the metabolism, functioning and/or development of the stamen cell and
2) a sterility promoter capable of directing expression of the male-sterility DNA selectively in stamen cells of the plant, the male-sterility DNA being in the same transcriptional unit as, and under the control of, the sterility promoter.
The male-sterility genotype in the maintainer plant cell of this invention preferably comprises, especially in the male-sterility locus, at least one second marker gene which comprises at least:
3) a second marker DNA encoding a second marker RNA and/or protein or polypeptide which, when present at least in the second specific tissue or specific cells of the plant, renders the plant easily separable from other plants which do not contain the second marker RNA, protein or polypeptide encoded by the second marker DNA at least in the second specific tissue or specific cells and
4) a second marker promoter capable of directing expression of the second marker DNA at least in the second specific tissue or specific cells, the second marker DNA being in the same transcriptional unit as, and under the control of, the second marker promoter.

Also in accordance with this invention are provided the maintainer plants, the seeds of such plants, and plant cell cultures, all of which consist essentially of the cells of this invention.
Further in accordance with this invention are provided the maintainer gene and plasmids containing the maintainer gene, as well as bacterial host cells (e.g., E. coli or Agrobacterium) containing such plasmids.
Still further in accordance with this invention is provided a process for producing, preferably enlarging, a homogeneous population of male-sterile plants and their seed that contain a nuclear male-sterility gene in homozygous condition, the process comprising the step of crossing the male-sterile plants with the maintainer plants of this invention. The seed from the resulting male-sterile plants can be harvested and grown into the male-sterile plants. Hybrid seed can then be produced by crossing the male-sterile plants with male-fertile plants of another inbred parent line used as pollinators.
Yet further in accordance with this invention is provided a process for producing, preferably enlarging, a population of the maintainer plants, comprising the step of selfing the maintainer plants.

## Detailed Description of the Invention

A male-sterile plant of this invention is a plant of a given species with a nuclear male-sterility genotype.
A restorer plant of this invention is a plant of the same plant species containing, within its nuclear genome, a fertility-restorer gene that is able to restore the male-fertility in offspring which are obtained from a cross between the male-sterile plant and the restorer plant and which contain both the male-sterility genotype and the fertility-restorer gene.
A restored plant of this invention is a plant of the same species that is male-fertile and that contains, within its nuclear genome, the male-sterility genotype and the fertility-restorer gene.
A parent plant or parent of this invention is a plant that can be used for the production of hybrid seed. The female or seed parent plant is the parent from which the hybrid seed is harvested. For the purposes of this invention, the female parent will always be a male-sterile plant. The male or pollen parent is the parent that is used to fertilize the female parent. In many cases, the male parent will also be a restorer plant.
A line is the progeny of a given individual plant.
The male-sterility genotype of this invention is the genotype of at least one locus, preferably only one locus, in the nuclear genome of a plant (i.e., the male-sterility locus), the allelic composition of which can result in male-sterility in the plant. A male-sterility genotype can be endogenous to the plant, but it is generally preferred that it be foreign to the plant. Preferred foreign male-sterility genotypes are those in which the allele responsible for male-sterility contains a foreign male-sterility gene that comprises:

1) a male-sterility DNA encoding a sterility RNA and/or protein or polypeptide which, when produced or overproduced in a stamen cell of the plant, significantly disturbs the metabolism, functioning and/or development of the stamen cell and
2) a sterility promoter capable of directing expression of the male-sterility DNA selectively in stamen cells of the plant, the male-sterility DNA being in the same transcriptional unit as, and under the control of, the sterility promoter.

Such a male-sterility gene is always a dominant allele at a foreign male-sterility locus. The recessive allele corresponds to the absence of the male-sterility gene in the nuclear genome of the plant.

Preferred foreign male-sterility DNAs and sterility promoters that can be used in the male-sterility genes in female parent plants and maintainer plants of this invention have been described in EP 0,344,029. A particularly useful male-sterility DNA codes for Barnase (Hartley (1988) J.Mol. Biol. 202:913). Particularly useful sterility promoters are tapetum-specific promoters such as: the promoter of the TA29 gene of Nicotiana tabacum (EP 0,344,029) which can be used in tobacco, oilseed rape, lettuce, chicory, corn and other plant species; the PT72, the PT42 and PE1 promoters from rice, the sequences of which are given in SEQ ID no. 7, SEQ ID no. 8, and SEQ ID no. 9, respectively, of the Sequence Listing and which can be used in rice and other plant species (PCT application PCT/EP 92/00274); and the PCA55 promoter from corn, the sequence of which is given in SEQ ID No. 10, which can be used in corn and other plant species (PCT application PCT/EP 92/00275).

A preferred endogenous male-sterility genotype is one in which a recessive allele ("m") in homozygous condition (m/m) at a male-sterility locus produces male-sterility. At a male-sterility locus, male-fertility would otherwise be encoded by a corresponding dominant allele ("M"). Such a male-sterility genotype is known in many plant species (see Kaul (1988) supra; and 1992 issues of Maize Genetics Cooperation Newsletter, published by the Department of Agronomy and U.S. Department of Agriculture, University Of Missouri, Columbia, Missouri, U.S.A.). The DNA sequences in the nuclear genome of a plant corresponding to m and M alleles can be identified by gene tagging, i.e., by insertional mutagenesis using transposons, or by means of T-DNA integration (see, e.g., Wienand and Saedler (1987) In "Plant DNA Infectious Agents", Ed. by T.H.Hohn and J.Schell, Springer Verlag, New York, p. 205; Shepherd (1988) In "Plant Molecular Biology: a Practical Approach", IRL Press, p. 187; Teeri et al (1986) EMBO J. 5:1755).

Fertility-restorer DNAs and restorer promoters that can be used in the maintainer genes of this invention with a foreign male-sterility genotype have been described in EP 0,412,911. In this regard, fertility-restorer genes in which the fertility-restorer DNA encodes Barstar (Hartley (1988) J.Mol. Biol. 202:913) and is under control of tapetum-specific promoters, such as those described above as sterility promoters, are of particular use. In particular, it is believed that a fertility-restorer DNA coding for a mutant of Barstar, in which the cysteine residue at its position 40 is replaced by serine (Hartley (1989) TIBS 14:450), functions better in restoring the fertility in the restored plants of some species.

When an endogenous male-sterility genotype is homozygous for a recessive allele m, it is preferred that the fertility-restorer gene be the dominant allele M of that male-sterility genotype, preferably under the control of its own promoter. The DNA corresponding to such a dominant allele, including its natural promoter, can be isolated from the nuclear genome of the plant by means of gene tagging as described above.

The pollen-lethality DNAs that are used in the pollen-lethality genes of this invention preferably encode an RNA and/or a protein or polypeptide that, when expressed in microspores or pollen, significantly disrupts their metabolism, functioning and/or development. In this regard, the pollen-lethality DNAs can encode RNAs, proteins or polypeptides such as are encoded by the male-sterility DNAs described in EP 0,344,029. Of particular interest are male-sterility DNAs that encode ribonucleases (EP 0,344,029) such as RNase T1 from Aspergillus oryzae (Quaas et al (1988) Eur. J. Biochem. 173:617) or Barnase from Bacillus amyloliquefaciens (Hartley (1988) J.Mol.Biol. 202:913).

So that the pollen-lethality DNA is expressed selectively in microspores or pollen of the maintainer plant, it is preferred that the pollen-specific promoter, which controls the pollen-lethality DNA in the pollen-lethality gene, be a promoter capable of directing gene expression selectively in the microspores and/or pollen of the plant. Such a pollen-specific promoter can be an endogeneous promoter or a foreign promoter and can be from the nuclear genome or from the mitochondrial or chloroplast genome of a plant cell, but in any event, the pollen-specific promoter is foreign in the nuclear genome of the plant being transformed. Preferably the pollen-specific promoter causes the pollen-lethality DNA to be expressed only in the microspores and/or pollen, i.e., after meiosis of the microsporocytes in the anthers. The pollen-specific promoter can be selected and isolated in a well known manner from a plant species, preferably the plant species to be rendered male-sterile, so that the pollen-specific promoter directs expression of the pollen-lethality DNA selectively in the microspores and/or pollen so as to kill or disable the microspores and/or pollen in which the pollen-lethality gene is expressed. The pollen-specific promoter is preferably also selected and isolated so that it is effective to prevent expression of the pollen-lethality DNA in other tissues of the plant. For example, a suitable endogeneous pollen-specific promoter can be identified and isolated in a plant, to be rendered male-sterile, by :

1. searching for an mRNA which is only present in the plant during the development of its microspores and/or pollen;
2. optionally isolating the microspore- and/or pollen-specific mRNA;
3. preparing and isolating a cDNA from the microspore- and/or pollen-specific mRNA;
4. using this cDNA as a probe to identify regions in the plant genome which contain DNA coding for the corresponding microspore- and/or pollen-specific DNA or alternatively using inverse polymerase chain reactions for the geometric amplification of the DNA sequences which flank, upstream and downstream, a chosen core region of the genomic DNA corresponding to the sequence of the microspore- and/or pollen-specific cDNA; and
5. identifying the portion of the plant genome that is upstream (i.e. 5') from the DNA coding for the microspore- and/or pollen-specific mRNA and that contains the promoter of this DNA.

Examples of such pollen-specific promoters are well known (see MacCormick (1991) TIG 7:298). In this regard, Hamilton et al (1989) Sex. Plant Reprod. 2:208 describes a pollen-specific clone ("Zmg13") from maize inbred line W-22, and the use of the promoter sequences of the clone to direct pollen-specific expression in tobacco has been described by Guerrero et al (1990) Mol.Gen.Genet. 224:161). Other pollen-specific promoters that are likewise believed to be useful are: the promoter of the gene corresponding to the Nicotiana tabacum pollen-specific cDNA NTPc303 described by Weterings et al (1992) Plant Mol. Biol. 18:1101; and the promoter of the gene corresponding to the Brassica napus pollen-specific cDNA B54 described by Shen and Hsu (1992) Mol. Gen. Genet. 234:379.

If the fertility-restorer DNA in the fertility-restorer gene of the maintainer gene is also expressed in microspores and/or pollen at the same time as the pollen-lethality DNA is expressed (due for instance to the activity of the restorer promoter in microspores and/or pollen), it is preferred that the pollen-lethality DNA be different from the male-sterility DNA (the expression of which is intended to be prevented by expression of the fertility-restorer DNA of the maintainer gene). For example, if the male-sterility DNA encodes Barnase in the male-sterile plants to be maintained, the fertility-restorer DNA in the maintainer gene should encode Barstar. Thus, if the restorer promoter in the maintainer gene also directs expression of the fertility-restorer DNA in microspores and/or pollen and at the same time as the pollen-lethality DNA is expressed, the pollen-lethality DNA preferably should not encode Barnase but rather, for example, another RNAse such as RNAse T1.

First and second marker DNAs and first and second marker promoters that can be used in the first and second marker genes of this invention are also well known (EP 0,344,029; EP 0,412,911). In this regard, it is preferred that the first and second marker DNAs be different, although the first and second marker promoters may be the same.

The fertility-restorer gene, the male-sterility gene, the pollen-lethality gene, and the first and second marker genes in accordance with this invention are generally foreign DNA sequences, preferably foreign chimaeric DNA sequences. Such foreign DNA sequences are preferably provided with suitable 3' transcription regulation sequences and polyadenylation signals, downstream (i.e. 3') from their respective fertility-restorer DNA, male-sterility DNA, pollen-lethality DNA, and first and second marker DNAs. In this regard, either foreign or endogenous, transcription termination and polyadenylation signals suitable for obtaining expression of such DNA sequences can be used. For example, the foreign 3' untranslated ends of genes, such as gene 7 (Velten and Schell (1985) Nucl. Acids Res. 13:6998), the octopine synthase gene (De Greve et al (1982) J.Mol. Appl. Genet. 1:499; Gielen et al (1983) EMBO J. 3:835; Ingelbrecht et al (1989) The Plant Cell 1:671), the nopaline synthase gene of the T-DNA region of Agrobacterium tumefaciens Ti-plasmid (De Picker et al (1982) J.Mol. Appl. Genet. 1:561), the chalcone synthase gene (Sommer and Saedler (1986) Mol. Gen. Genet. 202: 429-434), and the CaMV 19S/35S transcription unit (Mogen et al (1990) The Plant Cell 2:1261-1272), can be used.

By "foreign" with regard to a gene or genotype of this invention is meant that the gene or genotype contains a foreign DNA sequence such as a male-sterility DNA, a fertility-restorer DNA, a pollen-lethality DNA, or a marker DNA and/or a foreign promoter such as a sterility promoter, a restorer promoter, a pollen-specific promoter or a marker promoter. By "foreign" with regard to any DNA sequence, such as a coding sequence or a promoter, in a gene or genotype of this invention is meant that such a DNA is not in the same genomic environment in a plant cell, transformed with such a DNA in accordance with this invention, as is such a DNA when it is naturally found in the cell of the plant, bacteria, animal, fungus, virus or the like, from which such a DNA originates. This means, for example, that a foreign fertility-restorer DNA, male-sterility DNA, pollen-lethality DNA, or marker DNA can be: 1) a nuclear DNA in a plant of origin; 2) endogenous to the transformed plant cell (i.e., from a plant of origin with the same genotype as the plant being transformed); and 3) within the same transcriptional unit as its own endogenous promoter and 3' end transcription regulation signals (from the plant of origin) in the foreign gene or genotype in the transformed plant cell; but 4) inserted in a different place in the nuclear genome of the transformed plant cell than it was in the plant of origin so that it is not surrounded in the transformed plant cell by the genes which surrounded it naturally in the plant of origin. Likewise, a foreign fertility-restorer DNA, male-sterility DNA, pollen-lethality DNA, or marker DNA can also, for example, be: 1) a nuclear DNA in a plant of origin; and 2) endogenous to the transformed plant cell; but 3) in the same transcriptional unit as a different (i.e., not its own) endogenous promoter and/or 3' end transcription regulation signals in a foreign chimaeric gene or genotype of this invention in a transformed plant cell. A foreign fertility-restorer DNA, male-sterility DNA, pollen-lethality DNA, or marker DNA can also, for example, be: 1) a nuclear DNA in a plant of origin; and 2) endogenous to the transformed plant cell; but 3) in the same transcriptional unit as a heterologous promoter and/or 3' end transcription regulation signals in a foreign chimaeric gene or genotype of this invention in a transformed plant cell. A foreign fertility-restorer DNA, a male-sterility DNA, pollen-lethality DNA, or marker DNA can also, for example, be heterologous to the transformed plant cell and in the same transcriptional unit as an endogenous promoter and/or 3' transcription regulation signals (e.g., from the nuclear genome of a plant with the same genotype as the plant being transformed) in a foreign chimaeric DNA sequence of this invention in a transformed plant cell. Preferably, each fertility-restorer DNA, male-sterility DNA, pollen-lethality DNA, and marker DNA of this invention is heterologous to the plant cell being transformed.

By "heterologous" with regard to a DNA, such as a fertility-restorer DNA, a male-sterility DNA, a pollen-lethality DNA, a marker DNA, a fertility-restorer promoter, a sterility promoter, a pollen-specific promoter or a marker promoter or any other DNA sequence in a gene or a genotype of this invention is meant that such a DNA is not naturally found in the nuclear genome of cells of a plant with the same genotype as the plant being transformed. Examples of heterologous DNAs include chloroplast and mitochondrial DNAs obtained from a plant with the same genotype as the plant being transformed, but preferred examples are chloroplast, mitochondrial, and nuclear DNAs from plants having a different genotype than the plant being transformed, DNAs from animal and bacterial genomes, and chromosomal and plasmidial DNAs from fungal, bacterial and viral genomes.

By "chimaeric" with regard to a foreign DNA sequence of this invention is meant that at least one of its coding sequences : 1) is not naturally found under the control of the promoter present in the foreign DNA sequence; and/or 2) is not naturally found in the same genetic locus as at least one of its associated marker DNAs. Examples of foreign chimaeric DNA sequences of this invention comprise: a pollen-lethality DNA of bacterial origin under the control of a pollen-specific promoter of plant origin; and a pollen-lethality DNA of plant origin under the control of a pollen-specific promoter of plant origin and in the same genetic locus as a marker DNA of bacterial origin.

By "endogenous" with respect to a gene or genotype of this invention is meant that it is not foreign.

The foreign genes and genotypes of this invention, such as the male-sterility gene and genotype, the fertility-restorer gene and the pollen-lethality gene, can be described like any other genotype: capital letters denote the presence of the foreign genes and genotypes (the dominant allele) while small letters denote their absence (the recessive allele). Hence, in this description of the invention, "S" and "s" will denote the respective presence and absence of the male-sterility gene, "R" and "r" will denote the respective presence and absence of the fertility-restorer gene, and "P" and "p" will denote the respective presence and absence of the maintainer gene.

For an endogeneous male-sterility genotype of this invention, "m" will denote the recessive allele, and "M" will denote the dominant allele. Thus, the recessive allele m in homozygous condition (m/m) at a male-sterility locus would result in male-sterility, and the dominant allele M, when present at a male-sterility locus either in homozygous or heterozygous condition, results in male-fertility.

The cell of a plant, particularly a plant capable of being infected with Agrobacterium such as most dicotyledonous plants (e.g. Brassica napus), can be transformed using a vector that is a disarmed Ti-plasmid containing the male-sterility gene and/or the fertility-restorer gene and/or the pollen-lethality gene and/or the maintainer gene and/or the marker gene(s) of this invention and carried by Agrobacterium. This transformation can be carried out using the procedures described, for example, in EP 0,116,718 and EP 0,270,822. Preferred Ti-plasmid vectors contain a foreign DNA sequence of this invention between the border sequences, or at least located to the left of the right border sequence, of the T-DNA of the Ti-plasmid. Of course, other types of vectors can be used to transform the plant cell, using procedures such as direct gene transfer (as described, for example, in EP 0, 233,247), pollen mediated transformation (as described, for example, in EP 0,270,356, PCT publication WO 85/01856, and US patent 4,684,611), plant RNA virus-mediated transformation (as described, for example, in EP 0,067,553 and US patent 4,407,956) and liposome-mediated transformation (as described, for example, in US patent 4,536,475). Cells of monocotyledonous plants, such as the major cereals including corn, rice, wheat, barley and rye, can be transformed as described in PCT application PCT/EP 91/02198. In case the plant to be transformed is corn, other recently developed methods can also be used such as, for example, the methods described for certain lines of corn by Fromm et al (1990) Bio/Technology 8:833, Gordon-Kamm et al (1990) Bio/Technology 2:603 and Gould et al (1991) Plant Physiol. 95:426. In case the plant to be transformed is rice, recently developed methods can also be used such as, for example, the method described for certain lines of rice by Shimamoto et al (1989) Nature 338:274, Datta et al (1990) Bio/Technology 8:736 and Hayashimoto et al (1990) Plant Physiol. 93:857.

The so-transformed cell can be regenerated into a mature plant, and the resulting transformed plant can be used in a conventional breeding scheme to produce more transformed plants with the same characteristics or to introduce the male-sterility gene, the fertility-restorer gene, the pollen-lethality gene, the marker genes and/or the maintainer gene of this invention in other varieties of the same or related plant species. Seeds obtained from such plants contain the gene(s) of this invention as a stable genomic insert.

The maintainer plant of this invention is of the same species as a male-sterile plant line and can be used for the maintenance of the male-sterile line, i.e. to maintain a homogeneous population of male-sterile plants and a stock of pure seed of the female parent. The maintainer plant of this invention is itself a plant in which male-fertility has been restored and the genome of which contains both a male-sterility genotype and, in the maintainer locus, a fertility-restorer gene of this invention.

If a plant line with a homozygous male-sterility genotype ($A^{m/m}$ or $A^{S/S}$) is available, a maintainer plant for the male-sterile line can be directly obtained by transforming a male-sterile plant of the line with the maintainer gene of this invention and then selecting those transgenic plants which are male-fertility restored plants and in which the maintainer gene is stably integrated in the nuclear genome so that the genetic locus of the male-sterility genotype and of the maintainer gene are unlinked and segregate independently.

If the male-sterility genotype is foreign to the plant line, alternative strategies can be followed. For example, the maintainer plant of the present invention can be obtained by: transforming a plant cell of the plant line (A) with the maintainer gene of this invention (P); and then regenerating, from the so-transformed plant cell, a transgenic plant containing, stably integrated in its genome, the maintainer gene. Such a transgenic plant ($A^{P/p}$) can then be crossed as a female parent with a plant $A^{S/s,R/r}$ of the same line, which contains at separate loci in its genome a male-sterility gene (S) and a corresponding fertility-restorer gene (R), both in heterozygous condition, but which lacks the maintainer gene. Thus, the cross is in fact: $A^{S/s,R/r,p/p}$ (male) x $A^{s/s,r/r,P/p}$ (female), and the offspring with the genotype $A^{S/s,r/r,P/p}$ (or hereinafter "$A^{S/s,P/p}$" for convenience) are selected and selfed. One eighth of the offspring that have the desired genotype ($A^{S/S,P/p}$) for a maintainer plant of this invention can then be selected. Another eighth of the offspring with the genotype ($A^{S/S,p/p}$) can be used as male-sterile plants to be maintained.

Isolation of plants with desired genotypes can be achieved by means of conventional testcrosses (see, e.g., Fehr (1987) supra), preferably supplemented by detection of the presence of specific genes at the DNA level, e.g., by means of amplification of DNA fragments by the polymerase chain reaction, by Southern blot analysis and/or by phenotypic analysis for the presence and expression of first or second marker genes of this invention.

The cross of a male-sterile plant containing a male-sterility genotype in homozygous condition ($A^{S/S}$ or $A^{m/m}$) with a maintainer plant of this invention ($A^{S/S,P/p}$ or $A^{m/m,P/p}$, respectively) results in a population of seeds that all contain the male-sterility genotype in homozygous condition ($A^{S/S}$ or $A^{m/m}$, respectively) because the maintainer gene is not transmitted through the pollen. This property can be used to advantage in maintaining the basic seed and in the multiplication of basic seed for the final production of parent seed.

The maintainer plants of this invention ($A^{S/S,P/p}$ or $A^{m/m,P/p}$) can themselves be maintained by selfing. The offspring of such selfing will consist of 50% male-fertile maintainer plants ($A^{S/S,P/p}$ or $A^{m/m,P/p}$, respectively) and 50% male-sterile plants containing the male-sterility genotype in homozygous condition ($A^{S/S}$ or $A^{m/m}$, respectively). If desired, the male-sterile plants can be removed either manually on the basis of the male-sterile phenotype or, if the maintainer gene comprises a suitable first marker gene, preferably a first marker gene whose expression confers herbicide resistance to the plant, by using the phenotypic expression of the first marker gene (e.g, by applying herbicide to the offspring so that male-sterile plants that lack the herbicide-resistance gene are killed while maintainer plants with the herbicide-resistance gene survive).

Thus, the maintainer plant of this invention can be easily used to maintain a homogeneous population of male-sterile plants. In this regard, basic seed of a female parent of a given plant species can be crossed with an appropriate male parent to produce hybrid seed. Also, the maintainer plant of this invention can be used economically to multiply the basic seed of a female parent of a given plant species, so as to obtain sufficient quantities of female parent seed that can be crossed with an appropriate male parent to produce desired quantities of hybrid seed.

A male-sterile line, that is maintained and multiplied by the use of the maintainer plants of this invention, can be used for the production of hybrid seed. In principle, the male-sterile line ($A^{S/S}$) can be crossed directly with another male parent line ($B^{S/S}$) to produce hybrid seed ($AB^{S/s}$). However, as all hybrid plants are male-sterile, no reproduction and no seed set will occur. This is not a problem if the seed is not the harvested product (e.g., with lettuce), but where seed is the harvested product (e.g., with corn and oilseed rape), male-fertility in the hybrid plants should be at least partially restored. This can be accomplished by crossing the male-sterile line with a male-fertile parent line (e.g., $B^{R/R}$) that is also a restorer line, i.e. that also contains a fertility-restorer gene (R). The hybrids produced ($AB^{S/s,R/r}$) will be fully male-fertile. Alternatively the male-sterile-line ($A^{S/S}$) can first be crossed with the male-fertile line ($A^{S/S}$) just prior to hybrid seed productions. This has the advantage of giving a further multiplication of the female parent line. The offspring ($A^{S/s}$) can then be crossed with a suitable male-fertile parent line ($B^{s/s,r/r}$) to produce hybrid seed that is 50% male-fertile. If hybrid seed with 100% male fertility is desired, the offspring can be crossed with a suitable restorer male parent line ($B^{s/s,R/R}$).

In the case of a male-sterile line in which male-sterility is due to an endogeneous male-sterility genotype ($A^{m/m}$) at a male-sterility locus, hybrid seed can easily be produced by crossing the male-sterile line ($A^{m/m}$) with a line that is homozygous with respect to the endogenous dominant (male-fertility) allele at that male-sterility locus ($B^{M/M}$). All hybrid offspring of this cross will have the genotype $AB^{M/m}$ and will be fertile.

The maintainer plants of this invention can also be used as pollinator (i.e., male-fertile) plants in a cross with wild-type plants ($A^{s/s,p/p}$) of the same inbred line. The progeny of this cross will all be male-sterile and heterozygous for the male-sterility genotype ($A^{S/s,p/p}$). The progeny can therefore be used directly for hybrid seed production by crossing with a pollinator plant line B ($B^{s/s,p/p}$). This scheme only requires a male-sterilization of the wild-type plants, for example by manually removing the anthers (e.g., in corn) or by using a male gametocide.

Of course, by using the maintainer plants of this invention to maintain a homogeneous population of plants that are homozygous with respect to a male-sterility allele (whether dominant or recessive) that is encoded in the nuclear genome, the maintainer plants acquire many of the characteristics of plants of a cytoplasmic male-sterile line. However, such plants do not have one of the major disadvantages of cytoplasmic male-sterile plants, namely the cytoplasmic uniformity of the various male-sterile lines which, in corn, has led to serious problems (see Craig (1977) In "Corn and Corn Improvement", G.F. Sprague, ed., American Society of Agronomy, Inc., Publisher, p. 671).

Thus, the maintainer gene of this invention, when introduced into a particular line of a plant species, can always be introduced into any other line by backcrossing, but since the maintainer gene can only be transmitted through an egg, it will always be associated with the cytoplasm of the line in which it was initially introduced. However, since a maintainer plant line is only used for maintenance of a male-sterile line and not as a female parent for hybrid seed production, the hybrid seed will always contain the cytoplasm of the female parent, as desired.

The following Examples illustrate this invention. Unless otherwise indicated, all experimental procedures for manipulating recombinant DNA were carried out by the standardized procedures described in Sambrook et al (1989) "Molecular Cloning: A Laboratory Manual, Second Edition", Cold Spring Harbor Laboratory Press, N.Y. USA. All polymerase chain reactions ("PCR") were performed under conventional conditions, using the Vent™ polymerase (Cat. No. 254L - Biolabs New England, Beverley, MA 01915, U.S.A.) isolated from Thermococcus litoralis (Neuner et al (1990) Arch. Microbiol. 153:205-207). Oligonucleotides were designed by the methods described by Kramer and Fritz (1968) Methods in Enzymology 154:350 and synthesized by the phosphoramidite method (Beaucage and Caruthers (1981) Tetrahedron Letters 22:1859) on an applied Biosystems 380A DNA synthesizer (Applied Biosystems B.V., Maarssen, Netherlands).

The following bacterial strains and plasmids, used in the Examples, are available from the Deutsche Sammlung für Mikroorganismen und Zellkulturen ("DSM"), Mascheroder Weg 1B, Braunschweig, Germany:

| Bacterial strain | plasmid | DSM No | Date of Deposit |
|---|---|---|---|
| E. coli WK6 | pMa5-8 | DSM 4567 | May 3, 1988 |
| E. coli WK6 | pMc5-8 | DSM 4566 | May 3, 1988 |

In the Examples, reference will be made to the following Figure and Sequence Listing:

Figure

Figure 1: Ten-step procedure to obtain corn (e.g. H99) maintainer plants of the invention

Sequence Listing

| | |
|---|---|
| SEQ ID no. 1 | genomic DNA comprising the promoter of the Zml3 gene from Zea mays |
| SEQ ID no. 2 | sequence of plasmid "pVE144" |
| SEQ ID no. 3 | sequence of plasmid "pVE108" |
| SEQ ID no. 4 | sequence of oligonucleotide "MDB80" |
| SEQ ID no. 5 | sequence of oligonucleotide "MDB81" |
| SEQ ID no. 6 | sequence of oligonucleotide "MDB82" |
| SEQ ID No. 7 | genomic DNA comprising the anther specific promoter "PT72" from rice |
| SEQ ID No. 8 | genomic DNA comprising the anther specific promoter "PT42" from rice |
| SEQ ID No. 9 | genomic DNA comprising the anther specific promoter "PE1" from rice |
| SEQ ID No. 10 | genomic DNA comprising the anther specific promoter "PCA55" from corn |
| SEQ ID No. 11 | Oligonucleotide Zm13OLI2 |
| SEQ ID No. 12 | Oligonucleotide Zm130LI1 |
| SEQ ID No. 13 | Oligonucleotide Zm130LI5 |
| SEQ ID No. 14 | Oligonucleotide BX0L2 |
| SEQ ID No. 15 | Oligonucleotide TA29SBX0L2 |
| SEQ ID No. 16 | Oligonucleotide PTA290L5 |
| SEQ ID No. 17 | EcoRI-HindIII fragment of pTS218 carrying the maintainer gene. |

Examples

Example 1 : Isolation of the pollen-specific promoter of the Zm13 gene from maize.

A pollen-specific cDNA from Zea mays inbred line W-22, designated as "Zmc13", has been isolated and characterized by Hanson et al (1989) The Plant Cell 1:173. The corresponding genomic clone, designated as "Zmg13", containing substantial portions of the 5' flanking region has been isolated and characterized by Hamilton et al (1989) Sex. Plant Reprod. 2:208 (see also Hamilton et al (1992) Plant Mol. Biol. 18:211). The complete sequence of Zmg13 is shown in SEQ ID no. 1, and its promoter region will hereinafter be referred to as the "Zm13 promoter".

A corresponding promoter region from corn inbred line H99 was isolated as follows. Genomic DNA of inbred line H99 was prepared as described by Dellaporta et al (1983) Plant Mol. Biol. Reports 1:19-21. Using the genome as a substrate, a 1471 bp fragment was amplified by PCR using the oligonucleotides MDB80 and MDB82, the sequences of which are shown in SEQ ID no. 4 and SEQ ID no. 6, respectively. MDB80 corresponds to nucleotides 8 to 28 of Zmg13, while MDB82 is complementary to nucleotides 1458 to 1478 of Zmg13. Then, the purified amplified 1471 bp fragment was used as a substrate for the amplification by PCR of a 1422 bp fragment, using the oligonucleotides MDB80 and MDB81. MDB81 is complementary to nucleotides 1409 to 1429 of Zmg13, and its sequence is shown in SEQ ID no. 5. By using MDB81, a NcoI site is created in the amplified 1422 bp fragment at the ATG translation initiation codon.

The 1422 bp fragment is then ligated in an SmaI site of pGEM2 (Promega Corporation, Madison, Wisconsin 53711, U.S.A.), yielding plasmid pMDB13, and the fragment is sequenced (Maxam and Gilbert (1980) Meth. Enzymol. 65:499). The pollen-specific promoter of the Zm13 gene of corn inbred line H99 is obtained from pMDB13 as a EcoRV-NcoI fragment.

The Zm13 promoter is also cloned as follows. Genomic DNA of Zea mays line H99 is prepared as described above. Using the genomic DNA as a substrate, the following two fragments are amplified by means of PCR: 1) a 875 bp fragment is amplified using the oligonucleotides MDB80 (SEQ ID No. 4) and ZM130LI2 (which is complementary to nucleotides 859 to 882 of Zmg13 and which sequence is given in SEQ ID No. 11); and 2) a 661 bp fragment is amplified using the oligonucleotides Zm13OLI1 (which corresponds to nucleotides 767 to 791 of Zmg13 and which sequence is given in SEQ ID No. 12) and Zm13OLI5 (which is partially complementary to nucleotides 1397 to 1423 of Zmg13 and which sequence is given in SEQ ID No. 13). The 875 bp fragment, corresponding to the upstream region of the Zm13 promoter, is cloned into the SmaI site of pGEM2, yielding plasmid pTS204. The 661 bp fragment, corresponding to the downstream region of the Zm13 promoter, is digested with NcoI and cloned into plasmid pJB66 (Botterman and Zabeau (1987) DNA 6:583) digested with EcoRV and NcoI, yielding plasmid pTS203. Both fragments partly overlap and share a BstXI site in the region of overlap. Ligation of the 567 bp EcoRV-BstXI fragment of pTS204 and the 638 bp BstXI-NcoI fragment of pTS203 results in a 1205 bp fragment corresponding to the Zm13 promoter. This 1205 bp fragment, as cloned from line H99, is sequenced, and its sequence is found to be identical to the corresponding fragment of Zmg13 from line W-22 as given in SEQ ID No.1 except at position 276 (G in W-22 is T in H99), 410 (G in) W-22 is A in H99), and 1205-1206 (GC in W-22 is GGC in H99, thus corresponding to a 1 nucleotide insertion), numberings being as in SEQ ID No. 1.

Example 2 : Construction of plant transformation vectors comprising a maintainer gene that contain DNA encoding Barstar under the control of the TA29 promoter and DNA encoding Barnase under the control of the Zm13 promoter.

The 1205 bp EcoRV-NcoI fragment of pMDB13 is ligated to the large EcoRI-SmaI fragment of plasmid pVE144 and to the 739 bp EcoRI-NcoI fragment of pVE108, yielding plasmid pGSJVR1. Plasmid pVE144, the sequence of which is shown in SEQ ID no. 2, is a plasmid derived from plasmid pUC18 (Yanisch-Perron et al (1985) Gene 33:103) and containing DNA encoding neomycin phosphotranferase (neo) under the control of the 35S3 promoter (EP 0,359,617) from Cauliflower Mosaic Virus isolate CabbB-JI (Hull and Howell (1978) Virology 86:482) and DNA encoding the Barstar (Hartley (1988) J.Mol.Biol. 202:913) under the control of the tapetum-specific promoter of the TA29 gene of Nicotiana tabacum (EP 0,344,029; Seurinck et al (1990) Nucleic Acids Res. 18:3403). Plasmid pVE108, the sequence of which is shown in SEQ ID no. 3, is a plasmid derived from pUC18 and containing DNA encoding phosphinothricin acetyl transferase (bar) (EP 0,242,236) under the control of the 35S3 promoter and DNA encoding Barnase (Hartley (1980) supra) under the control of the TA29 promoter. The resulting plasmid, pGSJVR1 (which is subsequently renamed "pTS210"), is a pUC18-derived plasmid that contains a maintainer gene of this invention comprising: DNA encoding Barnase as the pollen-lethality DNA, the Zm13 promoter as the pollen-specific promoter, DNA encoding Barstar as the fertility-restorer DNA, the TA29 promoter as the restorer promoter, neo as the first marker DNA and the 35S3 promoter as the first marker promoter.

pTS210 is also obtained as follows. The 0.9 kb BstXI-SacI fragment of pTS204 is ligated to the large BstXI-SacI fragment of pTS203, yielding plasmid pTS206. The 1.47 BglII-NcoI kb fragment ofpTS206 is then ligated to the large NcoI-BglII fragment of pVE108, yielding plasmid pTS207. Finally, the 1.9 kb EcoRV-Eco-RI fragment of pTS207 is ligated to the large Eco-RI-SmaI fragment of pVE144, yielding plasmid pTS210.

A plasmid pTS218, which differs from pTS210 by carrying the bar gene as a selectable marker gene, is also obtained as follows:

- a 255 bp DNA fragment, designated as bxx and carrying the translation initiation site of the PTA29-barstar gene, is obtained by PCR using pVE144 as a template and oligonucleotides BXOL2 (SEQ ID No. 14) and TA29SBXOL2 (SEQ ID No. 15) as primers.

- a 492 bp DNA fragment is prepared by PCR using pVE108 and bxx as a template and oligonucleotides PTA290L5 (SEQ ID No. 16) and BXOL2 as primers. This 492 bp fragment is digested with AsnI and BspEI, and a 274 bp fragment is purified on gel and ligated to the 6.28 kb fragment of pVE144 which was digested with BspEI and partially digested with AsnI. The resulting plasmid is designated as pVEK144 and carries the PTA29-barstar-3'nos chimeric gene with an optimized translational initiation context.
- pVEK144 is digested with MunI and HindIII, and the 3.7 kbp fragment is isolated and ligated to the 1.7 kbp MunI-HindIII fragment of pVE108, yielding plasmid pVEB144 which carries the PTA29-barstar-3'nos and the P35S-bar-3'nos chimeric genes.
- theEcoRI-HindIII fragment of pVEB144, containing the two chimeric genes, is ligated to the large EcoRI-HindIII fragment of pUCNew2, yielding plasmid pVEC144. pUCNew2 is derived from pUC19 as described in WO 92/13956.
- finally, the large EcoRI-SmaI fragment of pVEC144 is ligated to the 1.9 bp EcoRV-EcoRI fragment of pTS207, yielding plasmid pTS218.

Plasmid pTS218 carries three chimeric genes, i.e., PTA29-barstar-3'nos (with optimized translational initiation context), P35S-bar-3'nos, and PZM13-barnase-3'nos. The EcoRI-HindIII fragment of pTS218 carrying these three chimeric genes is presented in the sequence listing as SEQ ID No. 17.

All steps of vector construction involving fragments of the barnase DNA, such as pVE108, pVE144, and ptS210, are carried out in E. coli strain MC1061 containing the cointegrate plasmid R702::pMc5BS which is obtained as follows. Plasmid pMc5BS, containing the barstar gene (encoding an inhibitor of barnase) under the control of the tac promoter (De Boer et al (1983) Proc. Natl. Acad. Sci. USA 80:21), is constructed by: cloning the EcoRI-HindIII fragment of plasmid pMT416 (Hartley (1988) supra) into the EcoRI and HindIII sites of plasmid pMc5-8 (DSM 4566); and then deleting the sequence starting with the initiation codon of the phoA signal sequence and ending with the last nucleotide before the translation initiation codon of the barstar-coding region by means of a looping-out mutagenesis procedure as generally described by Sollazo et al (1985) Gene 37:199.

Plasmid R702 is from Proteus mirabilis and can replicate in E.coli (Villarroel et al (1983) Mol. Gen. Genet. 189:390). Plasmid R702::pMc5BS is obtained by cointegration through illegitimate recombination between pMc5BS and R702, mediated by transposable elements present on R702 (Leemans (1982) "Technieken voor het gebruik van Ti-plasmieden van Agrobacterium tumefaciens als vectoren voor de genetic engineering van planten", Ph.D. Thesis Vrije Universiteit Brussel, Brussels, Belgium) and checked for induced expression of Barstar.

The use of E.coli (R702::pMc5BS) allows the construction, maintenance, amplification, and purification of plasmids containing the barnase DNA, such as pGSJVR1, without any lethal effect on the host due to accidental expression of the barnase DNA. However, because the Zm13 promoter is not expressed in E. coli, all steps of vector construction involving this promoter are also carried out in E. coli strain MC1061.

Example 3 : Transformation of corn with the maintainer gene of Example 2.

Zygotic immature embryos of about 0.5 to 1 mm are isolated from developing seeds of corn inbred line H99. The freshly isolated embryos are enzymatically treated for 1-2 minutes with an enzyme solution II (0.3% macerozyme (Kinki Yakult, Nishinomiya, Japan) in CPW salts (Powell & Chapman (1985) "Plant Cell Culture, A Practical Approach", R.A. Dixon ed., Chapter 3) with 10% mannitol and 5 mM 2-[N-Morpholino] ethane sulfonic acid (MES), pH 5.6). After 1-2 minutes incubation in this enzyme solution, the embryos are carefully washed with N6aph solution (macro- and micro-elements of N6 medium (Chu et al (1975) Sci. Sin. Peking 18:659) supplemented with 6mM asparagine, 12 mM proline, 1 mg/l thiamine-HCl, 0.5 mg/l nicotinic acid, 100 mg/l casein hydrolysate, 100 mg/l inositol, 30 g/l sucrose and 54 g/l mannitol). After washing, the embryos are incubated in the maize electroporation buffer, EPM-KCl (80 mM KCl, 5 mM $CaCl_2$, 10 mM HEPES (N-2-hydroxyethylpiperazine-N'-2-ethanesulfonic acid) and 0.425 M mannitol, pH 7.2). Approximately 100 embryos in 200 µl EPM-KCl are loaded in each electroporation cuvette. About 20 µg of a plasmid DNA, pPGSJVR1 (of Example 2) linearized with EcoRI, is added per cuvette.

After 1 hour DNA incubation with the explants, the cuvettes are transferred to an ice bath. After 10 minutes incubation on ice, the electroporation is carried out: one pulse with a field strength of 375 V/cm is discharged from a 900 µF capacitor. The electroporation apparatus is as described by Dekeyser et al (1990) The Plant Cell 2:591. Immediately after electroporation, fresh liquid N6aph substrate is added to the explants in the cuvette, after which the explants are incubated for a further 10 minute period on ice.

Afterwards, the embryos are transferred to Mah1 VII substrate (macro- and micro-elements and vitamins of N6 medium supplemented with 100 mg/l casein hydrolysate, 6 mM proline, 0.5 g/l MES, 1 mg/l 2,4-dichlorophenoxyacetic acid (2,4-D) and 2% sucrose solidified with 0.75 g/l $MgCl_2$ and 1.6 g/l Phytagel (Sigma Chemical Company, St Louis, Mo. U.S.A.), pH 5.8) and supplemented with 0.2M mannitol. After 3 days, the embryos are transferred to the same substrate supplemented with 200 mg/l kanamycin. After approximately 14 days, the embryos are transferred to Mah1 VII substrate without mannitol, supplemented with kanamycin. The embryos are further subcultured on this selective substrate for approximately 2 months with subculturing intervals of about 3 weeks. The induced embryogenic tissue is carefully isolated and transferred to MS medium (Murashige and Skoog (1962) Physiol. Plant 15:473) supplemented with 5 mg/l 6-benzylaminopurine for line H99. The embryogenic tissue is maintained on this medium for approximately 14 days and subsequently transferred to MS medium without hormones and 6% sucrose for line H99. Developing shoots are transferred to 1/2 MS medium with 1.5% sucrose for further development to normal plantlets. These plantlets are transferred to soil and cultivated in the greenhouse.

In an analogous way, corn embryos are transformed with a fragment of pTS218 DNA which contains the maintainer gene and the chimeric P35S-bar-3'nos and which is obtained by digestion of the plasmid with EcoRI, XhoI and PstI and by purifying the longest fragment. Transformation and plant regeneration is as described in Example 5.

**Example 4 : Analysis of the transgenic corn plants of Example 3.**

Plants form Example 3 transformed with pGSJVR1 are analysed for the presence of the maintainer gene by means of PCR. DNA is prepared according to the protocol described by Dellaporta et al (1983) Plant Mol. Biol. Reporter 1:19, adapted for application to tissue amounts of about 10 to 20 mg. For each plant, such an amount of tissue is macerated in extraction buffer in a microfuge tube. Representative fragments of the maintainer gene are amplified using appropriate oligonucleotide probes.

Activity of the expression product of the first marker gene (i.e., neomycin phosphotransferase II (NPTII)) is assayed in plants as follows. Crude extracts are prepared by grinding plant tissue in extraction buffer (McDonnell et al (1987) Plant Molecular Biol. Reporter 5:380). The extracts are then subjected to non-denaturing polyacrylamide gel electrophoresis according to the procedure described by Reiss et al (1984) Gene 30:211. NPTII activity is then assayed by in situ phosphorylation of kanamycin using [gamma-32P]ATP as a substrate (McDonnell et al (1987) supra).

The plants that are found to be positive on both the PCR and NPTII assay are further analyzed by means of Southern hybridization. Genomic DNA is prepared from plant tissue according to the protocol described by Dellaporta et al (1983) supra, supplemented by a treatment with RNase to remove remaining RNA. A non-transformed H99 plant is used as a control. Samples of the DNA are digested with appropriate restriction enzymes and subjected to horizontal agarose electrophoresis. Southern transfer to Hybond N+ (Amersham International PLC, Amersham, United Kingdom) membranes by means of the "alkali blotting of DNA" protocol and the subsequent hybridization are performed as recommended by the manufacturer (Amersham Hybond-N+ leaflet). Suitable radioactive probes are prepared with the multi-prime DNA labelling kit (Amersham) according to the protocol supplied by the manufacturer which is derived from published procedures (Feinberg and Vogelstein (1983) Anal. Biochem. 132:6). The banding patterns show that at least the maintainer gene is integrated into the plant genomic DNA.

The PCR assays show that the maintainer gene is present. The NPTII assays show that the first marker DNA is expressed. The mature transformed plants can then be analyzed phenotypically to see whether the barstar DNA is expressed in tapetum cells and the barnase gene is expressed in pollen cells. Expression of barstar is determined by northern blotting of anther mRNA and by making testcrosses to determine the restoration in the progeny. Expression of the pollen-lethality gene is determined by cytological examination of the anther. In this regard, viable and nonviable mature pollen is determined by analyzing the staining of isolated pollen upon incubation for 30 minutes at 24°C in the following reaction mixture: 100 mM phosphate buffer pH 7.8, 100 mM Sodiumsuccinate and 1 mM NitroBlue Tetrazolium, followed by visual inspection of formazan precipitation in viable pollen. Alternative techniques for the differentiation between viable and nonviable mature pollen are those described for example by Alexander (1969) Stain Technology 44:117, and by Heslop-Harrison and Heslop-Harrison (1970) Stain Technology 45:115. The viability of microspores is determined by embedding flower buds in plastic at different developmental stages and subjecting the buds to histochemical staining with the succinate dehydrogenase assay, both as described by De Block and Debrouwer (1992) The Plant Journal 2:261.

Ultimately, the progeny of the plant transformed with the pollen-lethality gene is determined. None of the offspring obtained from a cross using this plant as a male parent have this gene, while 50% of the offspring obtained from a cross using this plant as a female parent possess the gene.

Plants from Example 3, transformed with pTS218 DNA, are analyzed in the same way, except that the expression product of the first marker gene, i.e., phosphinothricine acetyltransferase, is assayed by means of a PAT assay as described in Example 5.

Example 5 : Production of male-sterile corn plants.

Zygotic embryos of corn inbred line H99 were isolated, enzymatically treated, washed, and loaded in electroporation buffer as described in Example 3. Approximately 100 embryos in 200 µl EPM-RCl were loaded in each electroporation cuvette. About 20 µg of a plasmid DNA, pVE108 linearized with HindIII, was added per cuvette. pVE108 is a 5620 bp plasmid which contains: a chimaeric gene comprising the bar DNA (EP 242236), encoding phosphinothricin acetyl transferase (PAT) and conferring resistance to an herbicidal glutamine synthetase inhibitor such as phosphinothricin (PPT), under the control of the 35S3 promoter; and another chimaeric gene comprising the DNA coding for barnase (Hartley (1988) supra) under the control of the tapetum-specific promoter of the TA29 gene (EP 344029) of N. tabacum. The complete sequence of plasmid pVE108 is given in SEQ ID no. 4. All vector constructions involving DNA fragments comprising the barnase gene were carried out in E. coli strain MC1061 containing the plasmid R702::pMc5BS of Example 3. After a 1 hour DNA incubation with the explants, the cuvettes were transferred to an ice bath. After 10 minutes incubation on ice, the electroporation was carried out as described in Example 3. Immediately after electroporation, fresh liquid N6aph substrate was added to the explants in the cuvette, after which the explants were incubated for a further 10 minute period on ice.

Afterwards, the embryos from one electroporation experiment were transferred to Mah1 VII substrate supplemented with 0.2 M mannitol and 2 mg/l PPT. After approximately 14 days, the embryos were transferred to Mh1 VII substrate (Mah1 VII substrate of Example 3 but without proline and casein hydrolysate) supplemented with 2 mg/l PPT but without mannitol. After approximately 4 weeks, the embryos were subcultured for another month on Mh1 VII substrate supplemented with 10 mg/l PPT. The induced embryogenic tissue was carefully isolated and transferred to MS medium supplemented with 5 mg/l 6-benzylaminopurine. The embryogenic tissue was maintained on this medium for approximately 14 days and subsequently transferred to MS medium without hormones and sucrose. Developing shoots were transferred to 1/2 MS medium with 1.5% sucrose for further development to normal plantlets. These plantlets survived an in vitro spraying with doses of BASTA$^R$ (Hoechst AG, Frankfurt am Main, Germany) corresponding to 2 l/ha. These plantlets were then transferred to soil and cultivated in the greenhouse, and two of the transformed plantlets, designated RZM35-1 and RZM35-18, were further characterized.

The embryos from a second electroporation experiment were transferred to Mh1 VII substrate supplemented with 2 mg/l PPT and 0.2 M mannitol. After about 14 days, the embryos were transferred to Mh1 VII substrate supplemented with 2 mg/l PPT but without mannitol. After approximately another three weeks, the embryos were transferred to Mh1 VII substrate supplemented with 10 mg/l PPT but without mannitol. After another three weeks, the induced embryogenic tissue was carefully isolated and transferred to MS medium supplemented with 2 mg/l PPT and 5 mg/l 6-benzylaminopurine. The embryogenic tissue was maintained on this medium for approximately 14 days and subsequently transferred to MS medium without hormones, sucrose or PPT. Developing shoots were transferred to 1/2 MS medium with 1.5% sucrose for further development to normal plantlets. The resulting plantlets were transferred to soil and cultivated in the greenhouse, and three of the transformed plantlets, designated RZM34-1, RZM34-12, and RZM34-14, were further characterized.

RZM34-1, RZM34-12, RZM34-14, RZM35-1, and RZM35-18 were grown in the greenhouse. Activity of the expression product of the bar gene in leaves of the plants was assayed as follows in a "PAT assay". 100 mg of leaf tissue from each plant, together with 50 mg of acid-treated sea sand (Merck, Darmstadt, Germany) and 5 mg polyvinylpolypyrrolidone (PVPP), were ground in an Eppendorf tube with a glass rod in 50 µl of extraction buffer (25 mM Tris-HCL pH 7.5, 1 mM $Na_2$-EDTA (ethylenediaminetetraacetic acid disodium salt), 0.15 mg/ml phenylmethylsulfonylfluoride (PMSF), 0.3 mg/ml dithiothreitol (DTT), and 0.3 mg/ml bovine serum albumin). The extract was centrifuged in a microfuge for 5 minutes at 16000 rpm. The supernatant was recovered and diluted ten times with TE 25/1 (25 mM Tris-HCL pH 7.5, 1 mM $Na_2$-EDTA. To twelve µl of the diluted extract was then added: 1µl of 1 mM PPT in TE 25/1, 1 µl of 2 mM AcetylCoenzyme A in TE 25/1, and 2 µl of [14C]AcetylCoenzym A (60 mCi/mmol, 0.02 mCi/ml, [NEN Research Products, Dupont, Wilmington, Delaware, USA). The reaction mixture was incubated for 30 minutes at 37°C and spotted on a aluminium sheet silicagel 60 t.l.c. plate with concentrating zone (Merck). Ascending chromatography was carried out in a 3 to 2 mixture of 1-propanol and $NH_4OH$ (25% $NH_3$). 14C was visualized by overnight autoradiography (XAR-5 Kodak film). The tolerance to the herbicide BASTA$^R$ was tested by brushing a small area near the top of one leaf per plant with a 1% solution of the herbicide and observing the damage symptoms at and near the brushed sites. While RZM34-1, RZM35-1 and RZM35-18 showed no damage symptoms at all, RZM34-12 and RZM34-14 displayed slight browning and drying-out of the brushed site. RZM34-1, RZM34-12, RZM34-14, RZM35-1 and RZM35-18 were also shown to be male-sterile but otherwise phenotypically completely normal; female fertility, for instance, was normal. The spikelets of the male flowers were of about normal length but were very thin and appeared to be empty, and they never opened. A detailed analysis showed that the anthers were reduced to almost microscopic structures. This phenotype indicates not only that at least one copy of the barnase gene was expressed but also that it was selectively expressed in some or all of the tissues of the anthers.

Southern analysis showed RZM35-1 and RZM35-18 to have an identical integration pattern, with only one copy of plasmid pVE108 being present in the genome of each plant. A small part of the plasmid DNA sequence adjacent to the HindIII site (used for linearization prior to electroporation) seemed to be absent in the integrated copy. Southern analysis of RZM34-1, RZM34-12 and RZM34-14 showed that each of these plants probably has two or three copies of part or all of pVE108 integrated into its genome. The copies are most likely not inserted in a concatemer configuration.

Transformants RZM35-1 and RZM34-1 were pollinated with pollen from an untransformed H99 plant, and progeny plantlets were recovered. From the 35 plantlets recovered from RZM35-1, 16 (46%) scored positive in a PAT assay, while 19 (54%) were PAT negative. This proportion in the F1 progeny does not differ significantly from the 1:1 ratio expected under normal Mendelian segregation of one active copy of the chimaeric bar gene (X2 = 0.26).

From the 34 plantlets recovered from RZM34-1, 19 (56%) scored positive in a PAT assay, while 15 (44%) were PAT negative. This proportion in the F1 progeny does not differ significantly from the 1:1 ratio expected under normal Mendelian segregation, assuming that the transformed female parent had one active copy, or alternatively multiple active, but closely linked copies, of the chimaeric bar gene (X2 = 0.47).

Example 6 : Production of restorer corn plants.

Zygotic embryos of corn inbred line H99 were isolated, enzymatically treated, washed and loaded in electroporation buffer as described in Example 5. Approximately 100 embryos in 200 µl EPM-KCl were loaded in each electroporation cuvette. About 20 µg of a plasmid DNA, pVE144 linearized with HindIII, was added per cuvette. pVE144 is a 6555 bp plasmid which was described in Example 2.

The embryos were electroporated, and the transformed cells were selected, grown into callus, and regenerated as described in Example 3. Transgenic plants were analyzed for the presence of the fertility-restorer gene and the marker gene by means of Southern hybridization and PCR. The expression of the fertility-restorer gene is assayed by means of Northern blotting, and the expression of the marker gene is determined by NPTII assay as described in Example 3.

Example 7 : Production of maintainer corn plants and a male-sterile corn line and maintenance of the male-sterile corn line

Maintainer plants of this invention of corn line H99 are obtained as outlined in Figure 1. A plant of corn inbred line H99 with the male-sterility genotype $H99^{S/s,r/r,p/p}$, transformed with the male-sterility gene of Example 5, is crossed with plants with the genotype $H99^{s/s,R/r,p/p,}$, transformed with the fertility-restorer gene of Example 6. The progeny that have the genotype $H99^{S/s,R/r,p/p}$ are identified by PCR analysis for the presence of the S and R genes. These plants are selfed, yielding progeny with nine different genotypes. Two of these genotypes ($H99^{S/S,r/r}$ and $H99^{S/s,r/r}$) will develop into male-sterile plants, while all the other genotypes will develop into male-fertile plants. When these male-fertile plants are selfed, progeny analysis allows the identification of their genotype. Thus: a) the progeny of selfings of $H99^{S/S,R/R}$, $H99^{S/s,R/R}$, $H99^{s/s,R/R}$, $H99^{s/s,R/r}$ and $H99^{s/s,r/r}$ would all develop into male-fertile plants; b) selfings of $H99^{S/s,R/r}$ plants would produce progeny, of which 13 out of 16 would be male-fertile, and since the male-sterility gene is linked to the herbicide resistance gene, bar, 4 out of the 13 male-fertile plants would be sensitive to the herbicide BASTA$^R$ ; and c) selfings of $H99^{S/S,R/r}$ plants would produce progeny, of which 12 out 16 would be fertile (4 out of 16 would have the genotype $H99^{S/S,R/R}$ and 8 out of 16 would have the genotype $H99^{S/S,R/r}$), all of which would be resistant to the herbicide, and the male-sterile progeny of which (4 out of 16) would all be homozygous for the male-sterility gene ($H99^{S/S,r/r}$).

The homozygous male-sterile progeny ($H99^{S/S,r/r}$) of selfing (c) are then crossed with their male-fertile siblings, and only when the cross is with plants with the genotype $H99^{S/S,R/r}$ are the resulting plants 50% male-sterile (all with the genotype $H99^{S/S,r/r}$) and 50% male-fertile (all with the genotype $H99^{S/S,R/r}$. Indeed, the alternative cross between $H99^{S/S,r/r}$ and $H99^{S/S,R/R}$ would result in 100% male-fertile progeny plants.

Maintainer plants are selected by crossing the plant with the genotype $H99^{S/s,R/r,p/p}$ with a plant that is heterozygous for the maintainer gene of Example 2, i.e., ($H99^{s/s,r/r,P/p}$), using the latter plant as the female parent. The offspring with the genotype $H99^{S/s,r/r,P/p}$ are selected by means of testcrosses supplemented with PCR analysis of the progeny (which can be easily identified by PCR and Southern blotting for the presence of the S and P genes and the absence of the R gene). The selected fertile offspring are then selfed. One out of eight offspring have the desired genotype for a maintainer plant of this invention ($H99^{S/S,P/p}$) and can be further selected by means of testcrosses and PCR analysis of the progeny. Indeed, only plants with this genotype will produce 50% male-sterile offspring (all $H99^{S/S,p/p}$) and 50% male-fertile offspring (all $H99^{S/S,P/p}$), thus growing at once both the desired homozygous male-sterile line and the maintainer line of this invention. Testcrosses also include the pollination of wild type H99 plants with pollen of the progeny plants obtained from the selfing of $H99^{S/s,P/p}$ plants.

Homozygous male-sterile plants with the genotype $H99^{S/S,r/r,p/p}$ are then pollinated by maintainer plants ($H99^{S/S,r/r,P/p}$) of this invention. All progeny have the genotype $H99^{S/S,r/r,p/p}$, so that the male-sterile line is maintained, as desired.

Example 8 : Introduction of the male-sterility gene and the maintainer gene in inbred corn lines through classical breeding.

The male-sterility gene of Example 5 and the maintainer gene of Example 2 are transferred from corn inbred line H99 to another corn inbred line (A) by repeated backcrossings as follows. The maintainer plant $H99^{S/S,P/p}$ is crossed as a female parent with an untransformed plant of line A ($A^{s/s,p/p}$). The offspring with the genotype A-$H99^{S/s,P/p}$ are selected by screening, using PCR, for the presence of both the maintainer gene (P) and the male-sterility gene (S). These plants are then crossed again as female parents with $A^{s/s,p/p}$ plants, and the offspring that are heterozygous for both the P and S genes are again selected by PCR. This process of backcrossing is repeated until finally plants with the genotype $A^{S/s,P/p}$ are obtained. These plants are then selfed, and the progeny are analyzed in the same way as described in Example 7. In this way, male-sterile plants with the genotype $A^{S/S,p/p}$ and maintainer plants of this invention with the genotype $A^{S/S,P/p}$ are obtained.

SEQUENCE LISTING

(1) GENERAL INFORMATION:

(i) APPLICANT:

(A) NAME: PLANT GENETIC SYSTEMS N.V.
(B) STREET: Jozef Plateaustraat 22
(C) CITY: Ghent
(E) COUNTRY: Belgium
(F) POSTAL CODE (ZIP): 9000
(G) TELEPHONE: 32 91 358411
(H) TELEFAX: 32 91 240694
(I) TELEX: 11.361 Pgsgen

(ii) TITLE OF INVENTION: Maintenance of male-sterile plants

(iii) NUMBER OF SEQUENCES: 17

(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:

(A) APPLICATION NUMBER: US 07/899,072
(B) FILING DATE: 12-JUN-1992

(vi) PRIOR APPLICATION DATA:

(A) APPLICATION NUMBER: US 07/970,849
(B) FILING DATE: 03-NOV-1992

(2) INFORMATION FOR SEQ ID NO: 1:

(i) SEQUENCE CHARACTERISTICS:

(A) LENGTH: 2661 base pairs
(B) TYPE: nucleic acid
(C) STRANDEDNESS: double

(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: Zea mays
(B) STRAIN: inbred line W-22

(x) PUBLICATION INFORMATION:

(A) AUTHORS: Hamilton et al.,
(C) JOURNAL: Sex Plant Reprod.
(D) VOLUME: 2
(F) PAGES: 208-
(G) DATE: 1989

(xi) SEQUENCE DESCRIPTION: SEQ ID NO: 1:

AAAGACCCCG CTTGTCAGTG AATGTTGCTA TTCTAGCAAA GGGAAGGTAT TTTTTCGGAC 60

CTTCGGCGTA AAGCCTTCGT CCAGATCGCA ATCTAAATTT ATTATTTTGA ACAAATTAAT 120

ATTGCGAGGG GCTACTGTTG GGGACCTTCG GCATCCGAAG GTCCTCAAAA ACAGGATTTA 180

ATAGTGTTTC TGGAGTATAA TGTGTGAACA GATATCTTCG GACTCAAGTC AGGCATCACA 240

GTAGACCAGA ATAATACGAA GGTTGGTGAA GCGCCGAAGG TGTAAGCAGG AAAGCTTCGG 300

CAAGACAGCA GCAGTTGAAA CCGACTTAAA GATGAAAAGG CTATTTAGAC CTCAACAGAT 360

TACTATAGGT TTATTATTAA GTGTAAAGGG CATTAATGTA ATTTTGCACG GGCTACGTCC 420

CGTGCCTATA AATAGGTGAA CAGTATTCCC GTACTGTTCA CGCTGACTTG GCATTCGCTT 480

TTTGCGTCAC GCTTGTACTG TCATCTCATT CCTATTGAAG GTACACTTGT AATTCAACGA 540

TATTTCTGTT TGTACCTAAT AATAATATAT AATTGTTCAT GTTGTCTTTT ATATTCTTTA 600

TATTTCATCC TTCGTCATTG TTTAATGAAT TTATGAAGGT ACGTCCTTCA TAACCTTCGT 660

CCGTAAACCA TTATATCCTA AGGGAAATAA TGCTTCGAAG GACGAAGGAC CTTAACGATT 720

AATATTTTCT ATGTTGCCTT GTTCTTAACT CATAGCACTT GAGAACAAGT CTCCAACAGT 780

TTGGTTATTC CTATTCCACG TGGATTAGAT GAGATTTAGA TAAAATTAGA AATAATTTTG 840

ACTTACTAGG GATTTAAACC AACTCAGTCC CGTTCAATCC ACATGGATTG AGATTAAAAC 900

AACTATTGAG ATTTTATTGT ATCAACACTC AACACCGATG TGTTTTTATA ATACATCTTG 960

CGTGACATTT GTCCAAGTAC TATGCTAAAT ATGAGAAGCT GCCATTTAGT GATTCTATAT 1020

ACTATTCACT TATGGATACA TTTAACTGAT ACCGTTTTGT TGAGCGCGTC TTATTTAGTT 1080

TTACATAGCA GCATAGAAGA TTAGAAGTCG CAAATCCAAC TTTTGTGGAC CGCTGAAAAA 1140

CTCAACCAAA TTCGACATAT TTTTCACCTC CCCATGCCAC AAAACTAGGT CAAAACGGCT 1200

TTCTGCCGTC GGCCACTATT TCTACGGGCA GCCAGACAAA TCTTCGGGTC TCGCAGATTA 1260

TTTAAGGACA CCACAGGCTG CGTTACGAAA CCAGGCCAGA TTTGCCACCC TCGTCTCACC 1320

CTCCCTCCCT CACACAAATA ATAAGGAAAG GTCCCGCCCT TTTCCTCCGA CATCCACAAG 1380

GGGGGAGGGG AAAACACGTA CATTCACCCG GCGGCAATAA TGGCCTCGGT TCCGGCTCCG 1440

GCGACGACGA CCGCCGCCGT CATCCTATGC CTATGCGTCG TCCTCTCCTG TGCCGCGGCT 1500

GACGACCCGA ACCTCCCCGA CTACGTCATC CAGGGCCGCG TGTACTGCGA CACCTGCCGC 1560

GCCGGGTTCG TGACCAACGT CACCGAGTAC ATCGCGGGCG CCAAGGTGAG GCTGGAGTGC 1620

AAGCACTTCG GCACCGGCAA GCTCGAGCGC GCCATCGACG GGGTCACCGA CGCGACCGGC 1680

ACCTACACGA TCGAGCTCAA GGACAGCCAC GAGGAGGACA TCTGCCAGGT GGTGCTGGTG 1740

GCCAGCCCGC GCAAGGACTG CGACGAGGTC CAGGCGCTCA GGGACCGCGC CGGCGTCCTG 1800

CTCACCAGGA ACGTTGGCAT CTCCGACAGC CTGCGCCCCG CCAACCCGCT AGGCTACTTC 1860

AAGGACGTGC CGCTCCCCGT CTGCGCCGCG CTGCTCAAGC AGCTGGACTC GGACGACGAC 1920

```
GACGACCAGT AAACTATACC ACGGCGGCGT CGCGGACATG CTGCACAAAA CTACAACGAT    1980
ACAGAGCGAA CGCATGGCAT GGATAGCAGT ATCTACGGAA AGAAAAGGAA GAAAAGGAAA    2040
ATAAAAAATG TATCAGAGTG CTTGATTCAC TTGCTGCTGT CACCCATTCC CCGTTCTTAA    2100
CATAACATGT GGGCCGGCTT GGCCCAGGCA CAAGCCCATC TACGCATGGC CTACGGTCCG    2160
CTAAAATATA GCCCTAATTA TGAGCCGTGT TGTGCCGTCA CATGGATCGA TCCAGCGGCA    2220
TACGATACAA CCCACAATTA CTTATGTGTG ATGGGCCGGC CAAAAAAGCC TAAGATGTCG    2280
TAGTGTGCTA GACCGACTCA TATATATAAA ACATTAAAAC ATATTGTCGG GGACCATAAT    2340
TAGGGGTACC CTTAAGGCTC CTAATTCTCA GCTGGTAACC CTCATCAGCG TAAAGCTGCA    2400
AAGGCCTGAT GGGTGCGATT AAGTCAGGGA TCAGTCCATT CGAGGGACTC GATCACGCCT    2460
CGCCCGAGCC TAGCCTCGGA CAAGGGCAGC CGACCCCGGA GGATCTCCGT CTCGCCCGAG    2520
GCCCTCCTCC AGCGGCGAAC ATATTTCCGG CTCGCCCGAG GCCCTGTCTT CGCCAAGAAG    2580
CAACCCTGAC CAAATCGCCG CACCGACCGA CCAAATCGCA GGAGCATTTA ATGCAAAGGT    2640
GGCCTGACAC ATTTATCCTG A                                              2661
```

(2) INFORMATION FOR SEQ ID NO: 2:

(i) SEQUENCE CHARACTERISTICS:

(A) LENGTH: 6555 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iii) ANTI-SENSE: NO

(vi) ORIGINAL SOURCE:

(A) ORGANISM: plasmid pVE144 (replicable in E.coli)

(ix) FEATURE:

(A) NAME/KEY: -
(B) LOCATION: 1..396
(D) OTHER INFORMATION: /label= pUC18 /note= "pUC18 derived sequence"

(ix) FEATURE:

(A) NAME/KEY: -
(B) LOCATION: complement (397..751)
(D) OTHER INFORMATION: /label= 3'nos /note= "3' regulatory sequence containing the polyadenylation site derived from Agrobacterium T-DNA nopaline synthase gene"

(ix) FEATURE:

(A) NAME/KEY: -

(B) LOCATION: complement (752..1024)
(D) OTHER INFORMATION: /label= barstar /note= "coding region of the barstar gene of Bacillus amyloliquefaciens"

(ix) FEATURE:

(A) NAME/KEY: -
(B) LOCATION: complement (1025..1607)
(D) OTHER INFORMATION: /label= TA29 /note= "promoter derived from the TA29 gene of Nicotiana tabacum"

(ix) FEATURE:

(A) NAME/KEY: -
(B) LOCATION: 1608..2440
(D) OTHER INFORMATION: /label= 35S3 /note= "35S3 promoter sequence derived from cauliflower mosaic virus isolate CabbB-JI"

(ix) FEATURE:

(A) NAME/KEY: -
(B) LOCATION: 2441..3256
(D) OTHER INFORMATION: /label= neo /note= "coding region of the neomycine phosphotransferase gene of Tn5"

(ix) FEATURE:

(A) NAME/KEY: -
(B) LOCATION: 3257..4315
(D) OTHER INFORMATION: /label= 3'ocs /note= "3' regulatory sequence containing the polyadenylation site derived from Agrobacterium T-DNA octopine synthase gene"

(ix) FEATURE:

(A) NAME/KEY: -
(B) LOCATION: 4316..6555
(D) OTHER INFORMATION: /label= pUC18 /note= "pUC18 derived sequence"

(xi) SEQUENCE DESCRIPTION: SEQ ID NO: 2:

```
TCGCGCGTTT CGGTGATGAC GGTGAAAACC TCTGACACAT GCAGCTCCCG GAGACGGTCA    60
CAGCTTGTCT GTAAGCGGAT GCCGGGAGCA GACAAGCCCG TCAGGGCGCG TCAGCGGGTG   120
TTGGCGGGTG TCGGGGCTGG CTTAACTATG CGGCATCAGA GCAGATTGTA CTGAGAGTGC   180
ACCATATGCG GTGTGAAATA CCGCACAGAT GCGTAAGGAG AAAATACCGC ATCAGGCGCC   240
ATTCGCCATT CAGGCTGCGC AACTGTTGGG AAGGGCGATC GGTGCGGGCC TCTTCGCTAT   300
TACGCCAGCT GGCGAAAGGG GGATGTGCTG CAAGGCGATT AAGTTGGGTA ACGCCAGGGT   360
TTTCCCAGTC ACGACGTTGT AAAACGACGG CCAGTGAATT CGAGCTCGGT ACCCGGGGAT   420
CTTCCCGATC TAGTAACATA GATGACACCG CGCGCGATAA TTTATCCTAG TTTGCGCGCT   480
ATATTTTGTT TTCTATCGCG TATTAAATGT ATAATTGCGG GACTCTAATC ATAAAAACCC   540
ATCTCATAAA TAACGTCATG CATTACATGT TAATTATTAC ATGCTTAACG TAATTCAACA   600
GAAATTATAT GATAATCATC GCAAGACCGG CAACAGGATT CAATCTTAAG AAACTTTATT   660
```

GCCAAATGTT TGAACGATCT GCTTCGGATC CTCTAGACCA AGCTAGCTTG CGGGTTTGTG 720
TTTCCATATT GTTCATCTCC CATTGATCGT ATTAAGAAAG TATGATGGTG ATGTCGCAGC 780
CTTCCGCTTT CGCTTCACGG AAAACCTGAA GCACACTCTC GGCGCCATTT TCAGTCAGCT 840
GCTTGCTTTG TTCAAACTGC CTCCATTCCA AAACGAGCGG GTACTCCACC CATCCGGTCA 900
GACAATCCCA TAAAGCGTCC AGGTTTTCAC CGTAGTATTC CGGAAGGGCA AGCTCCTTTT 960
TCAATGTCTG GTGGAGGTCG CTGATACTTC TGATTTGTTC CCCGTTAATG ACTGCTTTTT 1020
TCATCGGTAG CTAATTTCTT TAAGTAAAAA CTTTGATTTG AGTGATGATG TTGTACTGTT 1080
ACACTTGCAC CACAAGGGCA TATATAGAGC ACAAGACATA CACAACAACT TGCAAAACTA 1140
ACTTTTGTTG GAGCATTTCG AGGAAAATGG GGAGTAGCAG GCTAATCTGA GGGTAACATT 1200
AAGGTTTCAT GTATTAATTT GTTGCAAACA TGGACTTAGT GTGAGGAAAA AGTACCAAAA 1260
TTTTGTCTCA CCCTGATTTC AGTTATGGAA ATTACATTAT GAAGCTGTGC TAGAGAAGAT 1320
GTTTATTCTA GTCCAGCCAC CCACCTTATG CAAGTCTGCT TTTAGCTTGA TTCAAAAACT 1380
GATTTAATTT ACATTGCTAA ATGTGCATAC TTCGAGCCTA TGTCGCTTTA ATTCGAGTAG 1440
GATGTATATA TTAGTACATA AAAAATCATG TTTGAATCAT CTTTCATAAA GTGACAAGTC 1500
AATTGTCCCT TCTTGTTTGG CACTATATTC AATCTGTTAA TGCAAATTAT CCAGTTATAC 1560
TTAGCTAGAT GGGGATCCTC TAGAGTCGAC CTGCAGGCAT GCAAGCTCCT ACGCAGCAGG 1620
TCTCATCAAG ACGATCTACC CGAGTAACAA TCTCCAGGAG ATCAAATACC TTCCCAAGAA 1680
GGTTAAAGAT GCAGTCAAAA GATTCAGGAC TAATTGCATC AAGAACACAG AGAAAGACAT 1740
ATTTCTCAAG ATCAGAAGTA CTATTCCAGT ATGGACGATT CAAGGCTTGC TTCATAAACC 1800
AAGGCAAGTA ATAGAGATTG GAGTCTCTAA AAAGGTAGTT CCTACTGAAT CTAAGGCCAT 1860
GCATGGAGTC TAAGATTCAA ATCGAGGATC TAACAGAACT CGCCGTGAAG ACTGGCGAAC 1920
AGTTCATACA GAGTCTTTTA CGACTCAATG ACAAGAAGAA AATCTTCGTC AACATGGTGG 1980
AGCACGACAC TCTGGTCTAC TCCAAAAATG TCAAAGATAC AGTCTCAGAA GACCAAAGGG 2040
CTATTGAGAC TTTTCAACAA AGGATAATTT CGGGAAACCT CCTCGGATTC CATTGCCCAG 2100
CTATCTGTCA CTTCATCGAA AGGACAGTAG AAAAGGAAGG TGGCTCCTAC AAATGCCATC 2160
ATTGCGATAA AGGAAAGGCT ATCATTCAAG ATGCCTCTGC CGACAGTGGT CCCAAAGATG 2220
GACCCCCACC CACGAGGAGC ATCGTGGAAA AAGAAGACGT TCCAACCACG TCTTCAAAGC 2280
AAGTGGATTG ATGTGACATC TCCACTGACG TAAGGGATGA CGCACAATCC CACTATCCTT 2340
CGCAAGACCC TTCCTCTATA TAAGGAAGTT CATTTCATTT GGAGAGGACA CGCTGAAATC 2400
ACCAGTCTCT CTCTATAAAT CTATCTCTCT CTCTATAACC ATGGATCCGG CCAAGCTAGC 2460
TTGGATTGAA CAAGATGGAT TGCACGCAGG TTCTCCGGCC GCTTGGGTGG AGAGGCTATT 2520

CGGCTATGAC TGGGCACAAC AGACAATCGG CTGCTCTGAT GCCGCCGTGT TCCGGCTGTC 2580
AGCGCAGGGG CGCCCGGTTC TTTTTGTCAA GACCGACCTG TCCGGTGCCC TGAATGAACT 2640
GCAGGACGAG GCAGCGCGGC TATCGTGGCT GGCCACGACG GGCGTTCCTT GCGCAGCTGT 2700
GCTCGACGTT GTCACTGAAG CGGGAAGGGA CTGGCTGCTA TTGGGCGAAG TGCCGGGGCA 2760
GGATCTCCTG TCATCTCACC TTGCTCCTGC CGAGAAAGTA TCCATCATGG CTGATGCAAT 2820
GCGGCGGCTG CATACGCTTG ATCCGGCTAC CTGCCCATTC GACCACCAAG CGAAACATCG 2880
CATCGAGCGA GCACGTACTC GGATGGAAGC CGGTCTTGTC GATCAGGATG ATCTGGACGA 2940
AGAGCATCAG GGGCTCGCGC CAGCCGAACT GTTCGCCAGG CTCAAGGCGC GCATGCCCGA 3000
CGGCGAGGAT CTCGTCGTGA CCCATGGCGA TGCCTGCTTG CCGAATATCA TGGTGGAAAA 3060
TGGCCGCTTT TCTGGATTCA TCGACTGTGG CCGGCTGGGT GTGGCGGACC GCTATCAGGA 3120
CATAGCGTTG GCTACCCGTG ATATTGCTGA AGAGCTTGGC GGCGAATGGG CTGACCGCTT 3180
CCTCGTGCTT TACGGTATCG CCGCTCCCGA TTCGCAGCGC ATCGCCTTCT ATCGCCTTCT 3240
TGACGAGTTC TTCTGAGCGG GACTCTGGGG TTCGAAATGA CCGACCAAGC GACGCCCAAC 3300
CTGCCATCAC GAGATTTCGA TTCCACCGCC GCCTTCTATG AAAGGTTGGG CTTCGGAATC 3360
GTTTTCCGGG ACGCCGGCTG GATGATCCTC CAGCGCGGGG ATCTCATGCT GGAGTTCTTC 3420
GCCCACCCCC TGCTTTAATG AGATATGCGA GACGCCTATG ATCGCATGAT ATTTGCTTTC 3480
AATTCTGTTG TGCACGTTGT AAAAAACCTG AGCATGTGTA GCTCAGATCC TTACCGCCGG 3540
TTTCGGTTCA TTCTAATGAA TATATCACCC GTTACTATCG TATTTTTATG AATAATATTC 3600
TCCGTTCAAT TTACTGATTG TACCCTACTA CTTATATGTA CAATATTAAA ATGAAAACAA 3660
TATATTGTGC TGAATAGGTT TATAGCGACA TCTATGATAG AGCGCCACAA TAACAAACAA 3720
TTGCGTTTTA TTATTACAAA TCCAATTTTA AAAAAAGCGG CAGAACCGGT CAAACCTAAA 3780
AGACTGATTA CATAAATCTT ATTCAAATTT CAAAAGGCCC CAGGGGCTAG TATCTACGAC 3840
ACACCGAGCG GCGAACTAAT AACGTTCACT GAAGGGAACT CCGGTTCCCC GCCGGCGCGC 3900
ATGGGTGAGA TTCCTTGAAG TTGAGTATTG GCCGTCCGCT CTACCGAAAG TTACGGGCAC 3960
CATTCAACCC GGTCCAGCAC GGCGGCCGGG TAACCGACTT GCTGCCCCGA GAATTATGCA 4020
GCATTTTTTT GGTGTATGTG GGCCCCAAAT GAAGTGCAGG TCAAACCTTG ACAGTGACGA 4080
CAAATCGTTG GGCGGGTCCA GGGCGAATTT TGCGACAACA TGTCGAGGCT CAGCAGGGGC 4140
TCGATCCCCT CGCGAGTTGG TTCAGCTGCT GCCTGAGGCT GGACGACCTC GCGGAGTTCT 4200
ACCGGCAGTG CAAATCCGTC GGCATCCAGG AAACCAGCAG CGGCTATCCG CGCATCCATG 4260
CCCCCGAACT GCAGGAGTGG GGAGGCACGA TGGCCGCTTT GGTCGACCTG CAGCCAAGCT 4320
TGGCGTAATC ATGGTCATAG CTGTTTCCTG TGTGAAATTG TTATCCGCTC ACAATTCCAC 4380

ACAACATACG AGCCGGAAGC ATAAAGTGTA AAGCCTGGGG TGCCTAATGA GTGAGCTAAC 4440
TCACATTAAT TGCGTTGCGC TCACTGCCCG CTTTCCAGTC GGGAAACCTG TCGTGCCAGC 4500
TGCATTAATG AATCGGCCAA CGCGCGGGGA GAGGCGGTTT GCGTATTGGG CGCTCTTCCG 4560
CTTCCTCGCT CACTGACTCG CTGCGCTCGG TCGTTCGGCT GCGGCGAGCG GTATCAGCTC 4620
ACTCAAAGGC GGTAATACGG TTATCCACAG AATCAGGGGA TAACGCAGGA AAGAACATGT 4680
GAGCAAAAGG CCAGCAAAAG GCCAGGAACC GTAAAAAGGC CGCGTTGCTG GCGTTTTTCC 4740
ATAGGCTCCG CCCCCCTGAC GAGCATCACA AAAATCGACG CTCAAGTCAG AGGTGGCGAA 4800
ACCCGACAGG ACTATAAAGA TACCAGGCGT TTCCCCCTGG AAGCTCCCTC GTGCGCTCTC 4860
CTGTTCCGAC CCTGCCGCTT ACCGGATACC TGTCCGCCTT TCTCCCTTCG GGAAGCGTGG 4920
CGCTTTCTCA ATGCTCACGC TGTAGGTATC TCAGTTCGGT GTAGGTCGTT CGCTCCAAGC 4980
TGGGCTGTGT GCACGAACCC CCCGTTCAGC CCGACCGCTG CGCCTTATCC GGTAACTATC 5040
GTCTTGAGTC CAACCCGGTA AGACACGACT TATCGCCACT GGCAGCAGCC ACTGGTAACA 5100
GGATTAGCAG AGCGAGGTAT GTAGGCGGTG CTACAGAGTT CTTGAAGTGG TGGCCTAACT 5160
ACGGCTACAC TAGAAGGACA GTATTTGGTA TCTGCGCTCT GCTGAAGCCA GTTACCTTCG 5220
GAAAAAGAGT TGGTAGCTCT TGATCCGGCA AACAAACCAC CGCTGGTAGC GGTGGTTTTT 5280
TTGTTTGCAA GCAGCAGATT ACGCGCAGAA AAAAAGGATC TCAAGAAGAT CCTTTGATCT 5340
TTTCTACGGG GTCTGACGCT CAGTGGAACG AAAACTCACG TTAAGGGATT TTGGTCATGA 5400
GATTATCAAA AAGGATCTTC ACCTAGATCC TTTTAAATTA AAAATGAAGT TTTAAATCAA 5460
TCTAAAGTAT ATATGAGTAA ACTTGGTCTG ACAGTTACCA ATGCTTAATC AGTGAGGCAC 5520
CTATCTCAGC GATCTGTCTA TTTCGTTCAT CCATAGTTGC CTGACTCCCC GTCGTGTAGA 5580
TAACTACGAT ACGGGAGGGC TTACCATCTG GCCCCAGTGC TGCAATGATA CCGCGAGACC 5640
CACGCTCACC GGCTCCAGAT TTATCAGCAA TAAACCAGCC AGCCGGAAGG GCCGAGCGCA 5700
GAAGTGGTCC TGCAACTTTA TCCGCCTCCA TCCAGTCTAT TAATTGTTGC CGGGAAGCTA 5760
GAGTAAGTAG TTCGCCAGTT AATAGTTTGC GCAACGTTGT TGCCATTGCT ACAGGCATCG 5820
TGGTGTCACG CTCGTCGTTT GGTATGGCTT CATTCAGCTC CGGTTCCCAA CGATCAAGGC 5880
GAGTTACATG ATCCCCCATG TTGTGCAAAA AAGCGGTTAG CTCCTTCGGT CCTCCGATCG 5940
TTGTCAGAAG TAAGTTGGCC GCAGTGTTAT CACTCATGGT TATGGCAGCA CTGCATAATT 6000
CTCTTACTGT CATGCCATCC GTAAGATGCT TTTCTGTGAC TGGTGAGTAC TCAACCAAGT 6060
CATTCTGAGA ATAGTGTATG CGGCGACCGA GTTGCTCTTG CCCGGCGTCA ATACGGGATA 6120
ATACCGCGCC ACATAGCAGA ACTTTAAAAG TGCTCATCAT TGGAAAACGT TCTTCGGGGC 6180
GAAAACTCTC AAGGATCTTA CCGCTGTTGA GATCCAGTTC GATGTAACCC ACTCGTGCAC 6240

CCAACTGATC TTCAGCATCT TTTACTTTCA CCAGCGTTTC TGGGTGAGCA AAAACAGGAA 6300
GGCAAAATGC CGCAAAAAAG GGAATAAGGG CGACACGGAA ATGTTGAATA CTCATACTCT 6360
TCCTTTTTCA ATATTATTGA AGCATTTATC AGGGTTATTG TCTCATGAGC GGATACATAT 6420
TTGAATGTAT TTAGAAAAAT AAACAAATAG GGGTTCCGCG CACATTTCCC CGAAAAGTGC 6480
CACCTGACGT CTAAGAAACC ATTATTATCA TGACATTAAC CTATAAAAAT AGGCGTATCA 6540
CGAGGCCCTT TCGTC 6555

(2) INFORMATION FOR SEQ ID NO: 3:

(i) SEQUENCE CHARACTERISTICS:

(A) LENGTH: 5620 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iii) ANTI-SENSE: NO

(vi) ORIGINAL SOURCE:

(A) ORGANISM: plasmid pVE108 (replicable in E.coli)

(ix) FEATURE:

(A) NAME/KEY: -
(B) LOCATION: 1..395
(D) OTHER INFORMATION: /label= pUC18 /note= "pUC18 derived sequence"

(ix) FEATURE:

(A) NAME/KEY: -
(B) LOCATION: complement (396..802)
(D) OTHER INFORMATION: /label= 3'nos /note= "3' regulatory sequence containing the polyadenylation site derived from the nopaline synthase gene from Agrobacterium T-DNA"

(ix) FEATURE:

(A) NAME/KEY: -
(B) LOCATION: complement (803..1138)
(D) OTHER INFORMATION: /label= barnase /note= "coding region of the barnase gene of Bacillus amyloliquefaciens"

(ix) FEATURE:

(A) NAME/KEY: -
(B) LOCATION: complement (1139..1683)
(D) OTHER INFORMATION: /label= TA29 /note= "sequence derived from tapetum specific promoter of Nicotiana tabacum"

(ix) FEATURE:

(A) NAME/KEY: -
(B) LOCATION: 1684..2516
(D) OTHER INFORMATION: /label= 35S3 /note= ""3553" promoter sequence derived from cauliflower mosaic virus isolate CabbB-JI"

(ix) FEATURE:

(A) NAME/KEY: -
(B) LOCATION: 2517..3068
(D) OTHER INFORMATION: /label= bar /note= "coding sequence of phosphinotricin acetyltransferase gene"

(ix) FEATURE:

(A) NAME/KEY: -
(B) LOCATION: 3069..3356
(D) OTHER INFORMATION: /label= 3'nos /note= "3' regulatory sequence containing the polyadenylation site derived from Agrobacterium T-DNA nopaline synthase gene"

(ix) FEATURE:

(A) NAME/KEY: -
(B) LOCATION: 3357..5620
(D) OTHER INFORMATION: /label= pUC18 /note= "pUC18 derived sequence"

(xi) SEQUENCE DESCRIPTION: SEQ ID NO: 3:

TCGCGCGTTT CGGTGATGAC GGTGAAAACC TCTGACACAT GCAGCTCCCG GAGACGGTCA 60
CAGCTTGTCT GTAAGCGGAT GCCGGGAGCA GACAAGCCCG TCAGGGCGCG TCAGCGGGTG 120
TTGGCGGGTG TCGGGGCTGG CTTAACTATG CGGCATCAGA GCAGATTGTA CTGAGAGTGC 180
ACCATATGCG GTGTGAAATA CCGCACAGAT GCGTAAGGAG AAAATACCGC ATCAGGCGCC 240
ATTCGCCATT CAGGCTGCGC AACTGTTGGG AAGGGCGATC GGTGCGGGCC TCTTCGCTAT 300
TACGCCAGCT GGCGAAAGGG GGATGTGCTG CAAGGCGATT AAGTTGGGTA ACGCCAGGGT 360
TTTCCCAGTC ACGACGTTGT AAAACGACGG CCAGTGAATT CGAGCTCGGT ACCCGGGGAT 420
CTTCCCGATC TAGTAACATA GATGACACCG CGCGCGATAA TTTATCCTAG TTTGCGCGCT 480
ATATTTTGTT TTCTATCGCG TATTAAATGT ATAATTGCGG GACTCTAATC ATAAAAACCC 540
ATCTCATAAA TAACGTCATG CATTACATGT TAATTATTAC ATGCTTAACG TAATTCAACA 600
GAAATTATAT GATAATCATC GCAAGACCGG CAACAGGATT CAATCTTAAG AAACTTTATT 660
GCCAAATGTT TGAACGATCT GCTTCGGATC CTCTAGAGNN NNCCGGAAAG TGAAATTGAC 720
CGATCAGAGT TTGAAGAAAA ATTTATTACA CACTTTATGT AAAGCTGAAA AAAACGGCCT 780
CCGCAGGAAG CCGTTTTTTT CGTTATCTGA TTTTTGTAAA GGTCTGATAA TGGTCCGTTG 840
TTTTGTAAAT CAGCCAGTCG CTTGAGTAAA GAATCCGGTC TGAATTTCTG AAGCCTGATG 900
TATAGTTAAT ATCCGCTTCA CGCCATGTTC GTCCGCTTTT GCCCGGGAGT TTGCCTTCCC 960
TGTTTGAGAA GATGTCTCCG CCGATGCTTT TCCCCGGAGC GACGTCTGCA AGGTTCCCTT 1020
TTGATGCCAC CCAGCCGAGG GCTTGTGCTT CTGATTTTGT AATGTAATTA TCAGGTAGCT 1080

TATGATATGT CTGAAGATAA TCCGCAACCC CGTCAAACGT GTTGATAACC GGTACCATGG 1140
TAGCTAATTT CTTTAAGTAA AAACTTTGAT TTGAGTGATG ATGTTGTACT GTTACACTTG 1200
CACCACAAGG GCATATATAG AGCACAAGAC ATACACAACA ACTTGCAAAA CTAACTTTTG 1260
TTGGAGCATT TCGAGGAAAA TGGGGAGTAG CAGGCTAATC TGAGGGTAAC ATTAAGGTTT 1320
CATGTATTAA TTTGTTGCAA ACATGGACTT AGTGTGAGGA AAAAGTACCA AAATTTTGTC 1380
TCACCCTGAT TTCAGTTATG GAAATTACAT TATGAAGCTG TGCTAGAGAA GATGTTTATT 1440
CTAGTCCAGC CACCCACCTT ATGCAAGTCT GCTTTTAGCT TGATTCAAAA ACTGATTTAA 1500
TTTACATTGC TAAATGTGCA TACTTCGAGC CTATGTCGCT TTAATTCGAG TAGGATGTAT 1560
ATATTAGTAC ATAAAAAATC ATGTTTGAAT CATCTTTCAT AAAGTGACAA GTCAATTGTC 1620
CCTTCTTGTT TGGCACTATA TTCAATCTGT TAATGCAAAT TATCCAGTTA TACTTAGCTA 1680
GATCCTACGC AGCAGGTCTC ATCAAGACGA TCTACCCGAG TAACAATCTC CAGGAGATCA 1740
AATACCTTCC CAAGAAGGTT AAAGATGCAG TCAAAAGATT CAGGACTAAT TGCATCAAGA 1800
ACACAGAGAA AGACATATTT CTCAAGATCA GAAGTACTAT TCCAGTATGG ACGATTCAAG 1860
GCTTGCTTCA TAAACCAAGG CAAGTAATAG AGATTGGAGT CTCTAAAAAG GTAGTTCCTA 1920
CTGAATCTAA GGCCATGCAT GGAGTCTAAG ATTCAAATCG AGGATCTAAC AGAACTCGCC 1980
GTGAAGACTG GCGAACAGTT CATACAGAGT CTTTTACGAC TCAATGACAA GAAGAAAATC 2040
TTCGTCAACA TGGTGGAGCA CGACACTCTG GTCTACTCCA AAAATGTCAA AGATACAGTC 2100
TCAGAAGACC AAAGGGCTAT TGAGACTTTT CAACAAAGGA TAATTTCGGG AAACCTCCTC 2160
GGATTCCATT GCCCAGCTAT CTGTCACTTC ATCGAAAGGA CAGTAGAAAA GGAAGGTGGC 2220
TCCTACAAAT GCCATCATTG CGATAAAGGA AAGGCTATCA TTCAAGATGC CTCTGCCGAC 2280
AGTGGTCCCA AAGATGGACC CCCACCCACG AGGAGCATCG TGGAAAAAGA AGACGTTCCA 2340
ACCACGTCTT CAAAGCAAGT GGATTGATGT GACATCTCCA CTGACGTAAG GGATGACGCA 2400
CAATCCCACT ATCCTTCGCA AGACCCTTCC TCTATATAAG GAAGTTCATT TCATTTGGAG 2460
AGGACACGCT GAAATCACCA GTCTCTCTCT ATAAATCTAT CTCTCTCTCT ATAACCATGG 2520
ACCCAGAACG ACGCCCGGCC GACATCCGCC GTGCCACCGA GGCGGACATG CCGGCGGTCT 2580
GCACCATCGT CAACCACTAC ATCGAGACAA GCACGGTCAA CTTCCGTACC GAGCCGCAGG 2640
AACCGCAGGA GTGGACGGAC GACCTCGTCC GTCTGCGGGA GCGCTATCCC TGGCTCGTCG 2700
CCGAGGTGGA CGGCGAGGTC GCCGGCATCG CCTACGCGGG CCCCTGGAAG GCACGCAACG 2760
CCTACGACTG GACGGCCGAG TCGACCGTGT ACGTCTCCCC CCGCCACCAG CGGACGGGAC 2820
TGGGCTCCAC GCTCTACACC CACCTGCTGA AGTCCCTGGA GGCACAGGGC TTCAAGAGCG 2880
TGGTCGCTGT CATCGGGCTG CCCAACGACC CGAGCGTGCG CATGCACGAG GCGCTCGGAT 2940

ATGCCCCCCG CGGCATGCTG CGGGCGGCCG GCTTCAAGCA CGGGAACTGG CATGACGTGG 3000
GTTTCTGGCA GCTGGACTTC AGCCTGCCGG TACCGCCCCG TCCGGTCCTG CCCGTCACCG 3060
AGATCTGATC TCACGCGTCT AGGATCCGAA GCAGATCGTT CAAACATTTG GCAATAAAGT 3120
TTCTTAAGAT TGAATCCTGT TGCCGGTCTT GCGATGATTA TCATATAATT TCTGTTGAAT 3180
TACGTTAAGC ATGTAATAAT TAACATGTAA TGCATGACGT TATTTATGAG ATGGGTTTTT 3240
ATGATTAGAG TCCCGCAATT ATACATTTAA TACGCGATAG AAAACAAAAT ATAGCGCGCA 3300
AACTAGGATA AATTATCGCG CGCGGTGTCA TCTATGTTAC TAGATCGGGA AGATCCTCTA 3360
GAGTCGACCT GCAGGCATGC AAGCTTGGCG TAATCATGGT CATAGCTGTT TCCTGTGTGA 3420
AATTGTTATC CGCTCACAAT TCCACACAAC ATACGAGCCG GAAGCATAAA GTGTAAAGCC 3480
TGGGGTGCCT AATGAGTGAG CTAACTCACA TTAATTGCGT TGCGCTCACT GCCCGCTTTC 3540
CAGTCGGGAA ACCTGTCGTG CCAGCTGCAT TAATGAATCG GCCAACGCGC GGGGAGAGGC 3600
GGTTTGCGTA TTGGGCGCTC TTCCGCTTCC TCGCTCACTG ACTCGCTGCG CTCGGTCGTT 3660
CGGCTGCGGC GAGCGGTATC AGCTCACTCA AAGGCGGTAA TACGGTTATC CACAGAATCA 3720
GGGGATAACG CAGGAAAGAA CATGTGAGCA AAAGGCCAGC AAAAGGCCAG GAACCGTAAA 3780
AAGGCCGCGT TGCTGGCGTT TTTCCATAGG CTCCGCCCCC CTGACGAGCA TCACAAAAAT 3840
CGACGCTCAA GTCAGAGGTG GCGAAACCCG ACAGGACTAT AAAGATACCA GGCGTTTCCC 3900
CCTGGAAGCT CCCTCGTGCG CTCTCCTGTT CCGACCCTGC CGCTTACCGG ATACCTGTCC 3960
GCCTTTCTCC CTTCGGGAAG CGTGGCGCTT TCTCAATGCT CACGCTGTAG GTATCTCAGT 4020
TCGGTGTAGG TCGTTCGCTC CAAGCTGGGC TGTGTGCACG AACCCCCCGT TCAGCCCGAC 4080
CGCTGCGCCT TATCCGGTAA CTATCGTCTT GAGTCCAACC CGGTAAGACA CGACTTATCG 4140
CCACTGGCAG CAGCCACTGG TAACAGGATT AGCAGAGCGA GGTATGTAGG CGGTGCTACA 4200
GAGTTCTTGA AGTGGTGGCC TAACTACGGC TACACTAGAA GGACAGTATT TGGTATCTGC 4260
GCTCTGCTGA AGCCAGTTAC CTTCGGAAAA AGAGTTGGTA GCTCTTGATC CGGCAAACAA 4320
ACCACCGCTG GTAGCGGTGG TTTTTTTGTT TGCAAGCAGC AGATTACGCG CAGAAAAAAA 4380
GGATCTCAAG AAGATCCTTT GATCTTTTCT ACGGGGTCTG ACGCTCAGTG GAACGAAAAC 4440
TCACGTTAAG GGATTTTGGT CATGAGATTA TCAAAAAGGA TCTTCACCTA GATCCTTTTA 4500
AATTAAAAAT GAAGTTTTAA ATCAATCTAA AGTATATATG AGTAAACTTG GTCTGACAGT 4560
TACCAATGCT TAATCAGTGA GGCACCTATC TCAGCGATCT GTCTATTTCG TTCATCCATA 4620
GTTGCCTGAC TCCCCGTCGT GTAGATAACT ACGATACGGG AGGGCTTACC ATCTGGCCCC 4680
AGTGCTGCAA TGATACCGCG AGACCCACGC TCACCGGCTC CAGATTTATC AGCAATAAAC 4740
CAGCCAGCCG GAAGGGCCGA GCGCAGAAGT GGTCCTGCAA CTTTATCCGC CTCCATCCAG 4800

```
TCTATTAATT GTTGCCGGGA AGCTAGAGTA AGTAGTTCGC CAGTTAATAG TTTGCGCAAC     4860
GTTGTTGCCA TTGCTACAGG CATCGTGGTG TCACGCTCGT CGTTTGGTAT GGCTTCATTC     4920
AGCTCCGGTT CCCAACGATC AAGGCGAGTT ACATGATCCC CCATGTTGTG CAAAAAAGCG     4980
GTTAGCTCCT TCGGTCCTCC GATCGTTGTC AGAAGTAAGT TGGCCGCAGT GTTATCACTC     5040
ATGGTTATGG CAGCACTGCA TAATTCTCTT ACTGTCATGC CATCCGTAAG ATGCTTTTCT     5100
GTGACTGGTG AGTACTCAAC CAAGTCATTC TGAGAATAGT GTATGCGGCG ACCGAGTTGC     5160
TCTTGCCCGG CGTCAATACG GGATAATACC GCGCCACATA GCAGAACTTT AAAAGTGCTC     5220
ATCATTGGAA AACGTTCTTC GGGGCGAAAA CTCTCAAGGA TCTTACCGCT GTTGAGATCC     5280
AGTTCGATGT AACCCACTCG TGCACCCAAC TGATCTTCAG CATCTTTTAC TTTCACCAGC     5340
GTTTCTGGGT GAGCAAAAAC AGGAAGGCAA AATGCCGCAA AAAAGGGAAT AAGGGCGACA     5400
CGGAAATGTT GAATACTCAT ACTCTTCCTT TTTCAATATT ATTGAAGCAT TTATCAGGGT     5460
TATTGTCTCA TGAGCGGATA CATATTTGAA TGTATTTAGA AAAATAAACA AATAGGGGTT     5520
CCGCGCACAT TTCCCCGAAA AGTGCCACCT GACGTCTAAG AAACCATTAT TATCATGACA     5580
TTAACCTATA AAAATAGGCG TATCACGAGG CCCTTTCGTC                           5620
```

(2) INFORMATION FOR SEQ ID NO: 4:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: oligonucleotide MDB80

(ix) FEATURE:

(A) NAME/KEY: -
(B) LOCATION: 1..21
(D) OTHER INFORMATION: /label= MCB80 /note= "oligonucleotide designated as MDB80"

(xi) SEQUENCE DESCRIPTION: SEQ ID NO: 4:

```
CCGCTTGTCA GTGAATGTTG C                                                 21
```

(2) INFORMATION FOR SEQ ID NO: 5:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: oligonucleotide MDB81

(ix) FEATURE:

(A) NAME/KEY: -
(B) LOCATION: 1..21
(D) OTHER INFORMATION: /label= MDB81 /note= "oligonucleotide designated as MDB81"

(xi) SEQUENCE DESCRIPTION: SEQ ID NO: 5:

```
CCGAGGCCAT GGTTGCCGCC G                                                     21
```

(2) INFORMATION FOR SEQ ID NO: 6:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: oligonucleotide MDB82

(ix) FEATURE:

(A) NAME/KEY: -
(B) LOCATION: 1..21
(D) OTHER INFORMATION: /label= MDB82 /note= "oligonucleotide designated as MDB82"

(xi) SEQUENCE DESCRIPTION: SEQ ID NO: 6:

ACGCATAGGC ATAGGATGAC G 21

(2) INFORMATION FOR SEQ ID NO: 7:

(i) SEQUENCE CHARACTERISTICS:

(A) LENGTH: 3627 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: Oryza sativa
(B) STRAIN: Akihikari

(ix) FEATURE:

(A) NAME/KEY: -
(B) LOCATION: 1..2845
(D) OTHER INFORMATION: /label= PT72 /note= "sequence comprising anther specific promoter PT72"

(ix) FEATURE:

(A) NAME/KEY: -
(B) LOCATION: 2733..2739
(D) OTHER INFORMATION: /label= TATA /note= "TATA Box"

(ix) FEATURE:

(A) NAME/KEY: -
(B) LOCATION: 2765
(D) OTHER INFORMATION: /note= "transcription initiation determined by primer extension"

(ix) FEATURE:

(A) NAME/KEY: -
(B) LOCATION: 2846..2848
(D) OTHER INFORMATION: /label= ATG /note= "ATG start of translation of rice T72 gene"

(xi) SEQUENCE DESCRIPTION: SEQ ID NO: 7:

```
GACAATACAT CAAGTAAATC AAACATTACA AATCAGAACC TGTCTAAGAA TCCATCTTAA    60
TTCAGAAAAA AACTCAGATT AGATGTTCAT GCTTCCACCA GAAGCAGGAA TGTGCAACCT   120
ACACTTCCTG TAATTTCCAT ACTACAATGT CCCCACTGAC CACTGTGCCT GATGCTCTAT   180
TAGAATACCA CATCCTCCAT GGCTCCATGT AAATGCATAT AAATTTGACT CTTTAAATTA   240
GTAACTACAA TTTAAAATTT ATCGAACATT GTTCAAATTT ATAAACAGTT TCCCCAAATT   300
TAGATGCTCC CAAATGTACA CAGCTACTAG TAAAGCACCA TCCAGTTTCA CCTGAACAGG   360
ACTGACATAA ATGTGTGAAA AGGGGACGTC ATTCCCCCAA ATACAACTGA ACAATCCTCC   420
ATCAGAACAT TCATTTGATT GACATTACTC GGAGAGATAC AGCTCGCAGG CACACGAGAT   480
TCTTCTGCCT TTCCAATTGC CACGAACCCA CATGTCACAC GACCAACCAA AAAGAGAGAA   540
TTTTTCTTTG CACAAACAAA AAGTGAGATT TTTTTTTCGC CACAAAGGTG CGAACTTTCT   600
TCTCTCTCCC ACTTTCCAAT CAAGAAACGA AGCACTCAAA CCAAGAACAA ACCAAGGAAG   660
GAGAGATCGC TCCCTCTCCC AGAGCAAACG AAAGGAGAGA ACTCAGATGG ATGCGAACTA   720
CTACCTTGCC TCTTTCCCCG GAGAAGCAGC GAAGGAGAAG AGCGCGATGC CGCCGCCGCC   780
GCCGCCTCCG GCAACCTCCG GCTCCGGCGA GTCCGCCTCC TCCTCCTCTC TCACCTCTCT   840
```

CTTCCCAACC GTGTGGTGTT CGAGAAGCTT TTATGCGAGC GACGTGCAGT GGAAGCGGTT 900
GCTCCCAAGT CAAACTGATG GAGACCACCT ACTATCTTCC TCTTGTTTTC TTCTGCTTTT 960
CTTTTCTTTA TCTTTTTTCT TTCATTTTAT TTTGAGCGAT GAACTTGAGA ACAGTTTGGT 1020
TGTGGGTTAA ATTAAACGGT GCAGAATTGC AAAGCTACGT CCTTTTCGTC TGATTAAGGT 1080
GGTATCAGAA TCCTAATCTG TTAGCTCAGC ATTTGTTTTT GTGTGTTTAA TTGGCCATGA 1140
CATCAGATGG TTCAGACCGG TGGCAGGTCT TCATCGGAGA GGAGAATGAG AGCAATGCAA 1200
GTTGCAAACA ACAAACAGGT CCTTCCAAAC GGGTTGGTTT CATTCCACAG AACAGGATAG 1260
CAACCAGAGC ACAAACCGTT CAACAATATA TATATATATA TATATATATA TATATATATA 1320
TATATATATA TATATATATG ATTTAAAATT ATATTACTAT TTTTAGGATA CGGAACTCTT 1380
AACACATGAA AATCTAAACA TTTTCAACCA ATCAGAACTA CTAGAAAGAT AATCTAACTA 1440
CTTCAAAATT TAAAATTTGA CAAATAAAAT AACTAGTTTT TTCTAAAGCT ATCTTCACTG 1500
GACAACTTAT GAATATTTAT ATTTATGAAG CGAGTACTCT CCTAGTACAT ATTACATATA 1560
TATTCTTCTT CTCATGAAAA ATTAACTTCT CGCTATAAAT CCGAACATAT ATTATGCGTA 1620
GCAAGTTGTT TTTTTTAACG GGTGGAGTAA TATTAGAGTA TTTAAATTCC TTCAAATTGC 1680
CATCCCTCTG GGACTTTGCT GCTGTTGTTC TTCCACGGTT GCTGTCAGTG TCACCCAGAT 1740
TTGCATCCTT TCCAGCTCGT AGCTACTGTT CTGCATGTAT TGGACTTGGA TTAAGATCAA 1800
ATGCAGTTGC TATTGTAACT GCACAATAGC AACTGCACAC AATCATGTCC ATTCGTTTTC 1860
AGATCCAACG GCTCTAGATG ACTGCTACAG TACATGCATA ATAGTACATC TCTGCTACAG 1920
TGTTTTTGCT GCAGTACCAC TTCATATCCT GGCCTTCCGT TCTAGATCAT GTGATGTACA 1980
TGTTTTTTTG AAACAACCCG CACAAGACAT TGATAGAGTA GGAAATGTGA TGTACATGTT 2040
AACGGCTTAA GTTACAGTTA CAATAACAAC TGCACAGGAT CTTGATCCAT TGGACTTGTA 2100
TAATATCTCA TCTCGTCGTT CCATTATCGT GGTAACAGTT GGCAACTTGG CATCCAGTGC 2160
TGGAAACTAT GCCGTGTGTA CATCAGGATC GTCCTTTTTG TTCAGTTCCA AGATAGAACA 2220
AGTCCAAAAG ATGGCCGTAG TTTTTTTAGT CACAGTGGAA GCTGACATAG CCGTGGAATA 2280
AGTTCTGCAC AAAAGTTGCC ATTCGAGATC AACTACTGGT AGTAGTAGTC ATCTTCTACC 2340
ACTGCGAATA TTCGAAGGGA CACAAAAAGA TCAACGAGTA AATTAGTTCA CCGGAAGACG 2400
ACACATTATC ACCACAAAAA GACTAAAAAC AAAAAGAAAT TGCCAGGCCA AAAAAGGCAA 2460
AAAAGAAAAA AAAAGATGGC ACGAGGCCCA GGGCTACGGC CCATCTTGTC GCCGGCCCAA 2520
CCGCGCGCGC GAAACGCTCT CGTCGGCTCT CGGCTCGCCG CGACGCGATG GAGAGTTCGC 2580
GCCGCGGCGC GCGCGCGCGT TCGGTGGCTC ACACGCTTGC GCCCTCGTCC TCCCGGCCGG 2640
CGCGGGCGCC GACCGCGCGT CCGCCGCATG CGCGCGGCGT AGGTGAGCAA CGCGGGCCTC 2700

```
GCCGCGCGCG CTCCCCTCCT TCGATCCCCT CCTATAAATC GAGCTCGCGT CGCGTATCGC    2760
CACCACCACC ACGACACACA CGCACGCACC GTGCAGGCAT CGACGACGAG CGAGAGCCCC    2820
TCGGCGGCAG AAGACACTCA CGGCGATGGC GGTGACGAGG ACGGCGCTGC TGGTGGTGTT    2880
GGTAGCGGGG GCGATGACGA TGACGATGCG CGGGGCGGAG GCGCAGCAGC CGAGCTGCGC    2940
GGCGCAGCTC ACGCAGCTGG CGCCGTGCGC GCGAGTCGGC GTGGCGCCGG CGCCGGGGCA    3000
GCCGCTGCCG GCGCCCCCGG CGGAGTGCTG CTCGGCGCTG GGCGCCGTGT CGCACGACTG    3060
CGCCTGCGGC ACGCTCGACA TCATCAACAG CCTCCCCGCC AAGTGCGGCC TCCCGCGCGT    3120
CACCTGCCGT AAGAAAACGA ATAAAATCGA TTTGCTATCT ATCGATGATT GTGTTTTTGT    3180
AGACTAAACT AAACCCCTAT TAATAATCAA CTAACCGATG AACTGATCGT TGCAGAGTGA    3240
TGGAGATGGT GTGCCAAGGT AATTGCGTTT GCTCGTGCGA GGATGAGAAG AGAAGATTGA    3300
ATAAGATGTT TGATGGCAAC AAGTCATCAG GCGATCCGAT CCCTGCAGCT ATGAATGGGA    3360
GTATACGTAG TAGTGGTCTC GTTAGCATCT GTGTGTCGCA TATGCACGCC GTGCGTGCCG    3420
TGTCTGTCCT GCTTGCTCTG CTGATCGTTC AATGAACGAC AAATTAATCT AACTCTGGAG    3480
TGACAAGTCG TTCGAGATAT ACTAATACTA CCATGTGCAG GGTCTTTCAA CCAAGGTTCA    3540
TGTTTTCCAC GAAAGCCGAT TGAAACGAAA CCGCGAAATT TTGATGCGAG ATGAAAGCAG    3600
ATTCCGAGTG AAATTTTAAA TGGTTTT                                        3627
```

(2) INFORMATION FOR SEQ ID NO: 8:

(i) SEQUENCE CHARACTERISTICS:

(A) LENGTH: 2370 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: Oryza sativa
(B) STRAIN: Akihikari

(ix) FEATURE:

(A) NAME/KEY: -
(B) LOCATION: 1..1808
(D) OTHER INFORMATION: /label= PT42 /note= "sequence comprising anther specific promoter PT42"

(ix) FEATURE:

(A) NAME/KEY: -
(B) LOCATION: 1748..1755

(D) OTHER INFORMATION: /label= TATA /note= "TATA Box"

(ix) FEATURE:

(A) NAME/KEY: -
(B) LOCATION: 1780
(D) OTHER INFORMATION: /note= "transcription initiation site determined by primer extension"

(ix) FEATURE:

(A) NAME/KEY: -
(B) LOCATION: 1809
(D) OTHER INFORMATION: /label= ATG /note= "ATG start of translation of rice T42 gene"

(xi) SEQUENCE DESCRIPTION: SEQ ID NO: 8:

```
GGCCATCACT GTCGGGTGCT GCGCCATGGA CATCACCGTC TCCTTCCTGC GCCGCCGTCG      60
CCGGTGAGCT CCAAGGCCGA AGCCTTCTTC CCCTCACGCC ACTACCTCTC TCTTCCCCAA     120
TTCCGGCCAA CGCCGTCCGT TGCCACAGCG CCACCTCCAC GCCATCCCAG AGCCCCGTGC     180
CGTGCCACCG GGTTCGCCTC CATCTCCTCT TGCCAACGCC GACGCTCGTC GCGGCAGCCA     240
TGCGCTGTCA CCGATGAACA CCGCCGCGCC ACAGCCATGG CAGAGCACGG CCAGGGAGCC     300
ATGGCTGCTC TGCCTCCTCC TCCTTCTCTC ACATCTGGTT GCAGCCGGAC CTAGTCGGCT     360
TATACAAATG GCCCATGGGC AAAATTGTCT TTTATGAAAG TTTCTCTCAC CGTTTCAGTC     420
GGAAATAATA AAATAATGGG AGGATTGTCC GCCAGCAAAT TACCATATTT TTTCGGTGTC     480
CAAGAGCAAA TACACGATCT TCGGGTGTTT CACAGCAAAG ACCACAATTT CTAAGTGTCC     540
TGTAACAAAT TTTGCCAATA AAAATTTAAA ACCAAAGGAG AAGACTGTAC ATGAAGAAAA     600
ACAAAGAGAA TGAAATTACA TAAGCTCAGG GGTTATAAAG TTGATTTATT TTTAGGATGA     660
AGGAAGTGTG TGAAAACAAT GGCCAATTGG GTGTCGGAAA ATATAACGTG CTTGCTAAAA     720
TGTCGTCCCC ATATCCTGTA GCTGATTATA GATAGACCCT GATGGTCAAG ATGCCCTGTA     780
CTGGATCGTG TTTCCATGCT TCATCTCCGC TTCTCTCAAG TACTCCCCGA ACTCACATAT     840
CTGGTGGGCT GGATCCACAG TAAGAAACAG TCAAACAACA CTCACTTCAT AGATAACCAA     900
TTGTTTAATT ATTCTTAGTC CCTTATCTTA TACTCCTAGT AAGTGCTTAA AAACTTGGTA     960
TAAATATCAA ATTTATCGTA CAATTACAAT ATAATTATAA CGTATACCAT GTAATTTTTA    1020
AAACTATTTT TAGATAAAAA AAATATGGTG ATGAGCAGCC GCAGCAGCGG ACGCCGAACC    1080
ACCTGCCGAA CATCACCAAG ATAGCGAGTC CTAAAAATTT TTAGTGTTCG TTTGCTGGGT    1140
TGGTAACTAA TTAAAAAAAA AGAGCGACTC ATTAGCTCAT AAATAATTAC GTATTAGCTA    1200
ATTTTTTTAA AAAATAAATT AATATAACTT ATAAAGCAGC TTTTGTATAA TTTTTTTTTT    1260
AAAAAAGTGT TGTTTAGCAG TTTTGGGAAG TGTGCCGAGG GAAAACGATG AGATGGGTTG    1320
GGGAAGGAGG GGGAAGAAGT GAAGAACACA GCAAATATAG GCAGCATCGT CCCGTACAGA    1380
```

TCAGGCTGCA ACCACGCCCC GCGGAGATAG TTAACGCGGC CCACGTTGTG CTATAGCCCG 1440
TCACTCTCGC GGGCCTCTCC AACCTCCAGT TTTTTTTCTA GCCCATCAGC TGATACGGGG 1500
CCTTCCCCCC ATGCAGGAGG ATGGCCCGCC ACGCGGTGTT TTGGGCCGTT CTCGCCGCGC 1560
GCGCCCGTGC CGATCCGGGA CTCATCCCAC GTGCCGCCTC GCCACCGCCG CCGCCGCCGC 1620
TGCTGCTCCG GCTGCCGGCT GGACCTTCAC GCTCACGCGC TCTCCCCTGC CCAACCACCA 1680
CGCAAACAAA CACGAAGTTC GCGCCGTCGA CCGGCTCCCC TCCTCCCCCG CGCGCATCGG 1740
ATCCCCCTAC ATAAACCCTC TCGCTCGCCA TCGCCATGGC AGCAACTCCC CTCCTCCACT 1800
AGACCACCAT GCACAGATCG ATGGCCTCTC AGGCGGTGGC GCCCCTCCTC CTCATCCTCA 1860
TGCTCGCGGC GGCGGCGGGG GGCGCGTCGG CGGCGGTGCA GTGCGGGCAG GTGATGCAGC 1920
TGATGGCGCC GTGCATGCCG TACCTCGCCG GCGCCCCCGG GATGACGCCC TACGGCATCT 1980
GCTGCGACAG CCTCGGCGTG CTCAACCGGA TGGCCCCGGC CCCCGCCGAC CGCGTCGCCG 2040
TCTGCAACTG CGTCAAGGAC GCCGCCGCCG GCTTCCCCGC CGTCGACTTC TCCCGCGCCT 2100
CCGCCCTCCC CGCCGCCTGC GGCCTCTCCA TCAGCTTCAC CATCGCCCCC AACATGGACT 2160
GCAACCAGTA AGTTCATTCA TTCTTTCTTA ACTCCAATTC AATTTATCCA TCACCTCGAC 2220
TTAAGCCTGA TTAAACTTAA CTTGTTCTTT GCATGCTTGC ACTATTGCAG GGTTACAGAG 2280
GAACTGAGAA TCTGAGAGCG TGAGGAATCG AGTTCATGTT GCATTTATCA TCAATCATCA 2340
TCGACTAGAT CAATAAATCG AGCAAAGCTT 2370

(2) INFORMATION FOR SEQ ID NO: 9:

(i) SEQUENCE CHARACTERISTICS:

(A) LENGTH: 2407 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: Oryza sativa
(B) STRAIN: Akihikari

(ix) FEATURE:

(A) NAME/KEY: -
(B) LOCATION: 1..2263
(D) OTHER INFORMATION: /label= PE1 /note= "sequence comprising anther specific promoter PE1"

(ix) FEATURE:

(A) NAME/KEY: -
(B) LOCATION: 2181..2187
(D) OTHER INFORMATION: /label= TATA /note= "TATA Box"

(ix) FEATURE:

(A) NAME/KEY: -
(B) LOCATION: 2211
(D) OTHER INFORMATION: /note= "transcription initiation site determined by primer extension"

(ix) FEATURE:

(A) NAME/KEY: -
(B) LOCATION: 2264..2266
(D) OTHER INFORMATION: /label= ATG /note= "ATG start of translation of E1 gene"

(xi) SEQUENCE DESCRIPTION: SEQ ID NO: 9:

```
TGATAGTGAC ATACTCACAT GCTTTGTCAA TTCAAGTATC AGTTCTTTTC ATATTGATTT     60
CTTAGTTGAT GAAAGTATAC ATATTTCTTG CCATCAATTC TTTTAGTAGG TACATTTGGA    120
CACTAGTGGT CAGGGTTGAA CTCTTAACTG GAGTCTCATC TGATTTGCTT ATCTGAGACT    180
GGGTTTGTGC AAATCCTGTC ATGAGGCAAG GTGGACTGTC AGTCCATGAC ACTTTGCTAC    240
TTCTATTAAG TTCTCGAAAT CTTTTCCAGT GTATGTCCGT TCTCTTTCAA ATGAATTATT    300
TATATGTTCT GACAGCCTCG CGGTGTACAT TTCATTTAAC TTTTGTCTTC ACAGGGCCTC    360
TTGGTATTTT GTTGAGCAGA TTGGAATCAA CCTTCTTGTA GAACTTCTTG ATGTCGTCGC    420
TACCCTTTGC AACTAGATGG TCAACTTCTG TCTTATATCT TTGGTACAAC ACTGGCAAAG    480
TGTGCGCGCA CAAGAATCCT GTGAAGTAAG AAATACAAAC TTGTCATTGT GAAAGTTTAG    540
CTTTATATGA TCTTGACTCT AAATTGTTTC TCCTCAGATC CTTCTGTGTG ATTGTTTTAT    600
TAAAATTTAA TATTTATCTG GAATACCTAC CAATATATAG TAGACTTGTC AAGCTGCAAG    660
AACTTCCAAT CGCCGACAAT ACCAATAGAG ATCCAACCAC CTTAATATCA TAAACAATCT    720
GATTGTTAGT CCAGAACTAT ATTGAGTAGT GAACAACAAT AGCACATTAA CATTATGAGG    780
ATTATTGGCT AACTCTGCAA TTCAATATTC TGATGCGTCT AATCTGGTCA ATTTTAGCGC    840
TCCAGAAAGA ATTGCACAAT CCTTGGACAA TGTTGGCACT GGAACTGTTG CATGTTTTTA    900
CATCTCTTAT TAACGTAGCA AAGGAGTAGA TTATTATGTA CCAGGAGAAA TCTCTTCAGA    960
TCCTTTCCAC ATGCAATGTC GTAAAGAACA GATACAGTGT ACGTTAGTTT GTAATGGACG   1020
GTCAATGCCA TTTCTCTGAA GGCATGTTCA GAGATGATGA TTTCTGGGAT CCTTGGAGGG   1080
GCCCTGAAAT TCGGAAACAG TTAGTTGAGT TTTAGTACCT AATGTCTTGC GTTATACTAC   1140
GTGAAATGCC ATTTCTGTAA GCTGAGTTTT CTACCATCTC CACAGGAAAT AAAGCTAATA   1200
CCTGTCCAAG AGTGGTGCGG CATTTGACCA AATGAAGATC ACAAGCATGG CAAGAATGGC   1260
AATCTGGCAA AGGAGCGGAA TTATATTGTA TTCTACTACA TCGAACAGGA ACCATATCAA   1320
```

TGTTGCCCCA GCAAGGACCC CCGCAGATAA GTTCCTGTTC TTCCACAGCA GAATATCCGC 1380

AACTGCATAG CTCCCAACAA TGAAATCCAA AACCACATCG GCTCAGAGAG AAGTTATGAT 1440

AAAAGGCACT AATTCTGAAT AATTTCCTAG AAAGCGAATA ATAATAGCAC ACCTTGACCT 1500

CCACCAAGAA GCTTGTGGAT CGACTTGTGC CCATGAAATG GCATTCTGAC ATTCTGGTCA 1560

CTGTCAGAAT CTCTCGGAAA ATGAGGAGGC ATAGCTTCGT GTGTGTATGT GTGTGGGATA 1620

TTACGCTGCT AAAACTTTGT GTTTCTGATC GATCTGGTTA GAGAGCATCG TCTTTATAAG 1680

CACTTAAAAA TGGTAGTATA ATCTCTCAAG GAGCCTATAC TGCCAAGGAA AGGATAGCTT 1740

GGCCTGTGGG GATTGAGCCG TTGAAGGGAA CAAACGAATA CAGTTACCTT ACCAGATGTT 1800

TGCCACGACA TGGGCAACGT CATTGCTAGA CCAAGAAGGC AAGAAGCAAA GTTTAGCTGT 1860

CAAAAAAGAT ATGCTAGAGG CTTTCCAGAA TATGTTCTAT CTCAGCCAGA CCAATGGGGG 1920

CAAAATTTAC TACTATTTGC CATACATTAA CCACGTAAAA GTCCTACACT CAACCTAACT 1980

GTTGAACGGT CCTGTTCTGG CCAACGGTGA GAATGCACCT AATGGACGGG ACAACACTTC 2040

TTTCACCGTG CTACTGCTAC ATCCTGTAGA CGGTGGACGC GTGAGGTGCT TTCGCCATGA 2100

CCGTCCTTGG TTGTTGCAGT CACTTGCGCA CGCTTGCACC GTGACTCACC TGCCACATTG 2160

CCCCCGCCGT CGCCGGCGCC TACAAAAGCC ACACACGCAC GCCGGCCACG ATAACCCATC 2220

CTAGCATCCC GGTGTCCAGC AAGAGATCCA TCAAGCCGTC GCGATGACGA CGAGGCCTTC 2280

TGTTTTTTCC ACCGTTGTCG CGGCGATCGC CATCGCCGCG CTGCTGAGCA GCCTCCTCCT 2340

CCTGCAGGCT ACCCCGGCCG CGGCCAGCGC GAGGGCCTCG AAGAAGGCTT CGTGCGACCT 2400

GATGCAG 2407

(2) INFORMATION FOR SEQ ID NO: 10:

(i) SEQUENCE CHARACTERISTICS:

(A) LENGTH: 2784 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: Zea mays

(ix) FEATURE:

(A) NAME/KEY: -
(B) LOCATION: 1..1179
(D) OTHER INFORMATION: /label= PCA55 /note= "region comprising the anther specific promoter and the leader sequence, PCA55"

(ix) FEATURE:

(A) NAME/KEY: -
(B) LOCATION: 1072
(D) OTHER INFORMATION: /label= TATA /note= "TATA Box"

(ix) FEATURE:

(A) NAME/KEY: -
(B) LOCATION: 1180..1596
(D) OTHER INFORMATION: /note= "presumed coding sequence of corn CA55 gene"

(xi) SEQUENCE DESCRIPTION: SEQ ID NO: 10:

```
TGGTATGCAT CAATAGAGCC GGAAGATGGT CTGGAGTAAG GACCTGGCAG TGTGATACGG     60
GAACTTGACA TCTGAATAGA TATTCTCCCT TGTCCCTCTG GTAAAAAAAA CTGTTGTCAC    120
ATTTGCCTTC GCTGTGACTT GGATGTATCA TGTATATCTT TGACCATTGA TATCTTGGTT    180
AATCAGACGG TGCATTACAA TCATGGCCTC ATTCATATAG GGTTTAGGGT TACCACGATT    240
GGTTTGCATA AGTAGTACCC CTCCGTTTCA AATTATGTCG TATTTTGATT TTTTAGATAC    300
ACTTTTTATA TAATTTTTTA TTTTAAATTA GGTGTTTTAT ATAATACGTA TCTAAGTGTA    360
TAATAAAATA TATGTATCTA AAAGCTGTAA TTTAGTATAA ATTAGAATGG TGTATATCTT    420
CAATGTATGA CAAATAATTT GAAATGGAGG AGGGTATGAA AAGCCAAAAC CTCCTAGAAT    480
ATGGAATGGA GGGAATACAT ACAAATTCTT TGCTTCAGTT AAAAGAAACG AGAAAAGGAG    540
GGGAATGGGG AATCGTACTT CAGTTTTTAC GAGTTTTCAT CAAACATGTA TGCACGTCTT    600
CCCTTGGTTG ATGCATCTTT TTGGCAAATC TTCGTTTAAT TGCGGCTTCT TTTTTATACC    660
GTTCGAAGGT TTTCGTCGTC AATGCTGAAA CTCCACTTTC ACCACCTTCG GTTGCATCTG    720
CTTGCTTTCA ATTCACCTCT AATTAGTCCA AGTGTTTCAT TGGACGAAGG TCCAAGTCCT    780
TCAGATCATC TCAATTTTCT TTGATCTGAA ACAACAATTT AAAACTGATT TTGTTACCTT    840
GACCTGTCGA AGACCTTCGA ACGAACGGTA CTGTAAAAAT ACTGTACCTC AGATTTGTGA    900
TTTCAATTCG ATTCGGGTCT CCTGGCTGGA TGAAACCAAT GCGAGAGAAG AAGAAAAAAT    960
GTTGCATTAC GCTCACTCGA TCGGTTACGA GCACGTAGTT GGCGCCTGTC ACCCAACCAA   1020
ACCAGTAGTT GAGGCACGCC CTGTTTGCTC ACGATCACGA ACGTACAGCA CTATAAAACA   1080
CGCAGGGACT GGAAAGCGAG ATTTCACAGC TCAAAGCAGC CAAAACGCAG AAGCTGCACT   1140
GCATATACAG AAGATACATC GAGCTAACTA GCTGCAGCGA TGTCTCGCTC CTGCTGCGTC   1200
GCCGTGTCGG TGCTTCTCGC TGTCGCCGCG ACAGCCAGCG CCACCGCGCC GGCATGGCTG   1260
CACGAGGAGC AGCACCTCGA GGAGGCCATG GCCACGGGCC CGCTGGTCGC AGAGGGTGCG   1320
AGGGTGGCGC CCTCCGCGTC CACCTGGGCT GCCGACAAGG CGTCGCCGGC GAGGCCGAGC   1380
GGCGGCATGG CCACGCAGGG CGACGACCAG AGCTCGTCGG GCGGCAGTGG CAGCAGCGGT   1440
```

```
GAGCACGGCA AGGCGGAGGG CGAGAAGCAG GGCAAGAGCT GCCTCACCAA GGAGGAGTGC    1500
CACAAGAAGA AGATGATCTG TGGCAAGGGC TGCACGCTCT CGGCGCACAG CAAGTGCGCC    1560
GCCAAGTGCA CCAAGTCCTG TGTCCCCACC TGCTAGGAGC CGAGGCCGGA GCTTGCCGGC    1620
GGCGAGACCT CGATCGATCG AGTGCTTCAC TTCACTTCTT TGTTATAGTT CTTGTGTGTT    1680
GCCGTTGCGT TGCGTTGCGT AGACGAAGGG AATAAGGAAG GGTAATTGGA TTACCTGTTC    1740
CAGATCTCTG TGTAAGCGTG TTGTCGTGAC AAGTCTTTTG ATCCAGAGCG AGGGATGGAT    1800
AGATCGCGCT CGCAGTTTTA ATTGCAATGC TAGTTCAATA TGTGTGCATC ATGTTGGCAA    1860
CTACATAGTC CAGATTCAAA CCGAGATCGC TGTTTAGCAT GCCAGCACAA TAATAACGGT    1920
ACAATCATAT TATATTTTAT ACAAATGCAC AATTTATCTC TAGAGATGTC AATGGGAAAT    1980
TCCTCATCGG GTTATATCAT CTCAGACTCA TCCCCATCAT ATTTGATTCA TCCTCATACT    2040
CATCCTCATA TCTATCATGA GTGCAAAACT CATTTCATAC CCATCTCTAT TTTGGTTTAG    2100
GGTCTCCATC CCTAATTAAG GGATAACTAG TACTAACAAC TAGCACAAAC TATCTAGATT    2160
TCAGATATCA CCACATTGAC AAACAATCAT CCATGAACTA TGATCCATTC ATCCATCCAT    2220
CAAAAAATAA ATCGGTATTT CGAGAACGAT AGAAGAAATG AAGTCGGCTC ACCTTTCTTG    2280
GTCACCATTT GAGTTTGTTG GTGCCTGAGA ATCCATGGTC GTCATCGTCG TCCTAGGGAT    2340
CGGCGGTGCT CCTCGTTGTT GGTAAAGTCG CCAGTGTGTA GTGCTAGCGC AACTGTCCAG    2400
GCGTGCAACG GTTGGCCGGC TGGAAAGGGC ATAGCGTATG GCTGGTTATT TTTAGGGTTT    2460
TGTTTTTTTA CTAATCTGCT AGTTGCCTTG CCATGTTGTC TTATTGGGCT AGGATCTAGG    2520
GCTTGTTACG CTGCTGTGTT GGGCTTGGTG TCCGGTTCAG CCTCAACTCA TTCATACAAA    2580
TCAGATTCAT ACAAAACAGG TATACACGTA TGAAATATCC ATGGATAATC AGGTTCGAAT    2640
TATTGTCCCC TAAACCCATA CACGTTTACC CAATGGATGG ATATTTTGTC TCATATCCAT    2700
ACACATGAGA CGATTTTTGT CCCATACCTG TGCTCTAATA GGAGAATTTC TCTCGGGATA    2760
GCGAGTATCG GATCCTCTAG AGTC                                           2784
```

(2) INFORMATION FOR SEQ ID NO: 11:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: oligonucleotide Zm13Oli2

(ix) FEATURE:

(A) NAME/KEY: -
(B) LOCATION: 1..24
(D) OTHER INFORMATION: /label= Zm13Oli2 /note= "oligonucleotide designated as Zm13Oli2"

(xi) SEQUENCE DESCRIPTION: SEQ ID NO: 11:

GTGGATTGAA CGGGACTGAG TTGG 24

(2) INFORMATION FOR SEQ ID NO: 12:

(i) SEQUENCE CHARACTERISTICS:

(A) LENGTH: 25 base pairs
(B) TYPE: nucleic acid
(C) STRANDEDNESS: single
(D) TOPOLOGY: linear

(ii) MOLECULE TYPE: DNA (genotaic)

(iii) HYPOTHETICAL: NO

(iii) ANTI-SENSE: NO

(vi) ORIGINAL SOURCE:

(A) ORGANISM: oligonucleotide Zm13Oli1

(ix) FEATURE:

(A) NAME/KEY: -
(B) LOCATION: 1..25
(D) OTHER INFORMATION: /label= Zm13Oli1 /note= "oligonucleotide designated as Zm13Oli1"

(xi) SEQUENCE DESCRIPTION: SEQ ID NO: 12:

AAGTCTCCAA GACTTTGGTT ATTCC 25

(2) INFORMATION FOR SEQ ID NO: 13:

(i) SEQUENCE CHARACTERISTICS:

(A) LENGTH: 31 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: Oligonucleotide Zm13Oli5

(ix) FEATURE:

(A) NAME/KEY: -
(B) LOCATION: 1..31
(D) OTHER INFORMATION: /label= Zm13Oli5 /note= "oligonucleotide designated as Zm13Oli5"

(xi) SEQUENCE DESCRIPTION: SEQ ID NO: 13:

GGATCCATGG TTGCCGCCGG GTGAATGTAC G 31

(2) INFORMATION FOR SEQ ID NO: 14:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: oligonucleotide BXOL2

(ix) FEATURE:

(A) NAME/KEY: -
(B) LOCATION: 1..24
(D) OTHER INFORMATION: /label= BXOL2 /note= "oligonucleotide designated as BXOL2"

(xi) SEQUENCE DESCRIPTION: SEQ ID NO: 14:

ACGGAAAACC TGAAGCACAC TCTC 24

(2) INFORMATION FOR SEQ ID NO: 15:

(i) SEQUENCE CHARACTERISTICS:

(A) LENGTH: 49 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: oligonucleotide TA29SBXOL2

(ix) FEATURE:

(A) NAME/KEY: -
(B) LOCATION: 1..49
(D) OTHER INFORMATION: /label= TA29SBXOL2 /note= "oligonucleotide designated as TA29SBXOL2"

(xi) SEQUENCE DESCRIPTION: SEQ ID NO: 15:

GTTTTTACTT AAAGAAATTA GCTACCATGA AAAAAGCAGT CATTAACGG 49

(2) INFORMATION FOR SEQ ID NO: 16:

(i) SEQUENCE CHARACTERISTICS:

(A) LENGTH: 27 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: oligonucleotide PTA290L5

(ix) FEATURE:

(A) NAME/KEY: -
(B) LOCATION: 1..27
(D) OTHER INFORMATION: /label= PTA29OL5 /note= "oligonucleotide designated as PTA29OL5"

(xi) SEQUENCE DESCRIPTION: SEQ ID NO: 16:

TGGCCATAAC TGAAATCAGG GTGAGAC 27

(2) INFORMATION FOR SEQ ID NO: 17:

(i) SEQUENCE CHARACTERISTICS:

(A) LENGTH: 4808 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:

(A) ORGANISM: EcoRI-HindIII fragment of plasmid pTS218

(ix) FEATURE:

(A) NAME/KEY: -
(B) LOCATION: complement (18..401)
(D) OTHER INFORMATION: /label= 3'nos /note= "3' regulatory sequence containing the polyadenylation site derived from Agrobacterium T-DNA nopaline synthase gene"

(ix) FEATURE:

(A) NAME/KEY: -
(B) LOCATION: complement (402..737)
(D) OTHER INFORMATION: /label= barnase /note= "coding region of the barnase gene of Bacillus amyloliquefaciens"

(ix) FEATURE:

(A) NAME/KEY: -
(B) LOCATION: complement (738..1944)
(D) OTHER INFORMATION: /label= PZM13 /note= "promoter region of the Zm13 gene of Zea mays"

(ix) FEATURE:

(A) NAME/KEY: -
(B) LOCATION: complement (1945..2281)
(D) OTHER INFORMATION: /label= 3'nos

(ix) FEATURE:

(A) NAME/KEY: -
(B) LOCATION: complement (2282..2554)
(D) OTHER INFORMATION: /label= barstar /note= "coding region of the barstar gene of Bacillus amyloliquefaciens"

(ix) FEATURE:

(A) NAME/KEY: -
(B) LOCATION: complement (2555..3099)
(D) OTHER INFORMATION: /label= PTA29 /note= "promoter region of the TA29 gene of Nicotiana tabacum"

(ix) FEATURE:

(A) NAME/KEY: -
(B) LOCATION: 3100..3932
(D) OTHER INFORMATION: /label= 35S3 /note= ""35S3" promoter sequence derived from cauliflower mosaic virus isolate CabbB-JI"

(ix) FEATURE:

(A) NAME/KEY: -
(B) LOCATION: 3933..4484
(D) OTHER INFORMATION: /label= bar /note= "coding region of the phosphinothricin acetyltransferase gene"

(ix) FEATURE:

(A) NAME/KEY: -
(B) LOCATION: 4485..4763
(D) OTHER INFORMATION: /label= 3'nos

(ix) FEATURE:

(A) NAME/KEY: -
(B) LOCATION: 2333..2356
(D) OTHER INFORMATION: /label= BXOL2 /note= "region corresponding to oligonucleotide BXOL2"

(ix) FEATURE:

(A) NAME/KEY: -
(B) LOCATION: complement (2538..2586)
(D) OTHER INFORMATION: /label= TA29SBXOL2 /note= "region complementary to oligonucleotide TA29SBXOL2"

(ix) FEATURE:

(A) NAME/KEY: -
(B) LOCATION: complement (2800..2823)
(D) OTHER INFORMATION: /label= PTA29OL5 /note= "region complementary to part of oligonucleotide PTA29OL5"

(xi) SEQUENCE DESCRIPTION: SEQ ID NO: 17:

```
GAATTCGAGC TCGGTACCCG GGGATCTTCC CGATCTAGTA ACATAGATGA CACCGCGCGC     60
GATAATTTAT CCTAGTTTGC GCGCTATATT TTGTTTTCTA TCGCGTATTA AATGTATAAT    120
TGCGGGACTC TAATCATAAA AACCCATCTC ATAAATAACG TCATGCATTA CATGTTAATT    180
ATTACATGCT TAACGTAATT CAACAGAAAT TATATGATAA TCATCGCAAG ACCGGCAACA    240
GGATTCAATC TTAAGAAACT TTATTGCCAA ATGTTTGAAC GATCTGCTTC GGATCCTCTA    300
GAGNNNNCCG GAAAGTGAAA TTGACCGATC AGAGTTTGAA GAAAAATTTA TTACACACTT    360
TATGTAAAGC TGAAAAAAAC GGCCTCCGCA GGAAGCCGTT TTTTTCGTTA TCTGATTTTT    420
GTAAAGGTCT GATAATGGTC CGTTGTTTTG TAAATCAGCC AGTCGCTTGA GTAAAGAATC    480
CGGTCTGAAT TTCTGAAGCC TGATGTATAG TTAATATCCG CTTCACGCCA TGTTCGTCCG    540
CTTTTGCCCG GGAGTTTGCC TTCCCTGTTT GAGAAGATGT CTCCGCCGAT GCTTTTCCCC    600
GGAGCGACGT CTGCAAGGTT CCCTTTTGAT GCCACCCAGC CGAGGGCTTG TGCTTCTGAT    660
TTTGTAATGT AATTATCAGG TAGCTTATGA TATGTCTGAA GATAATCCGC AACCCCGTCA    720
AACGTGTTGA TAACCGGTAC CATGGTTGCC GCCGGGTGAA TGTACGTGTT TTCCCCTCCC    780
CCCTTGTGGA TGTCGGAGGA AAAGGGCGGG ACCTTTCCTT ATTATTTGTG TGAGGGAGGG    840
AGGGTGAGAC GAGGGTGGCA AATCTGGCCT GGTTTCGTAA CGCAGCCTGT GGTGTCCTTA    900
AATAATCTGC GAGACCCGAA GATTTGTCTG GCTGCCCGTA GAAATAGTGG CCGACGGCCA    960
GAAAGCCGTT TTGACCTAGT TTTGTGGCAT GGGGAGGTGA AAAATATGTC GAATTTGGTT   1020
GAGTTTTTCA GCGGTCCACA AAAGTTGGAT TTGCGACTTC TAATCTTCTA TGCTGCTATG   1080
TAAAACTAAA TAAGACGCGC TCAACAAAAC GGTATCAGTT AAATGTATCC ATAAGTGAAT   1140
AGTATATAGA ATCACTAAAT GGCAGCTTCT CATATTTAGC ATAGTACTTG GACAAATGTC   1200
ACGCAAGATG TATTATAAAA ACACATCGGT GTTGAGTGTT GATACAATAA AATCTCAATA   1260
GTTGTTTTAA TCTCAATCCA TGTGGATTGA ACGGGACTGA GTTGGTTTAA ATCCCTAGTA   1320
AGTCAAAATT ATTTCTAATT TTATCTAAAT CTCATCTAAT CCACGTGGAA TAGGAATAAC   1380
CAAACTGTTG GAGACTTGTT CTCAAGTGCT ATGAGTTAAG AACAAGGCAA CATAGAAAAT   1440
ATTAATCGTT AAGGTCCTTC GTCCTTCGAA GCATTATTTC CCTTAGGATA TAATGGTTTA   1500
CGGACGAAGG TTATGAAGGA CGTACCTTCA TAAATTCATT AAACAATGAC GAAGGATGAA   1560
ATATAAAGAA TATAAAAGAC AACATGAACA ATTATATATT ATTATTAGGT ACAAACAGAA   1620
ATATCGTTGA ATTACAAGTG TACCTTCAAT AGGAATGAGA TGACAGTACA AGCGTGACGC   1680
AAAAAGCGAA TGCCAAGTCA GCGTGAACAG TACGGGAATA CTGTTCACCT ATTTATAGGC   1740
```

ACGGGACGTA GCCTGTGCAA AATTACATTA ATGCCCTTTA CACTTAATAA TAAACCTATA 1800
GTAATCTGTT GAGGTCTAAA TAGCCTTTTC ATCTTTAAGT CGGTTTCAAC TGCTGCTGTC 1860
TTGCCGAAGC TTTCCTGCTT ACACCTTAGG CGCTTCACCA ACCTTCGTAT TATTCTGGTC 1920
TACTGTGATG CCTGACTTGA GTCCGAAGAT GGGGATCTTC CCGATCTAGT AACATAGATG 1980
ACACCGCGCG CGATAATTTA TCCTAGTTTG CGCGCTATAT TTTGTTTTCT ATCGCGTATT 2040
AAATGTATAA TTGCGGGACT CTAATCATAA AAACCCATCT CATAAATAAC GTCATGCATT 2100
ACATGTTAAT TATTACATGC TTAACGTAAT TCAACAGAAA TTATATGATA ATCATCGCAA 2160
GACCGGCAAC AGGATTCAAT CTTAAGAAAC TTTATTGCCA AATGTTTGAA CGATCTGCTT 2220
CGGATCCTCT AGACCAAGCT AGCTTGCGGG TTTGTGTTTC CATATTGTTC ATCTCCCATT 2280
GATCGTATTA AGAAAGTATG ATGGTGATGT CGCAGCCTTC CGCTTTCGCT TCACGGAAAA 2340
CCTGAAGCAC ACTCTCGGCG CCATTTTCAG TCAGCTGCTT GCTTTGTTCA AACTGCCTCC 2400
ATTCCAAAAC GAGCGGGTAC TCCACCCATC CGGTCAGACA ATCCCATAAA GCGTCCAGGT 2460
TTTCACCGTA GTATTCCGGA AGGGCAAGCT CCTTTTTCAA TGTCTGGTGG AGGTCGCTGA 2520
TACTTCTGAT TTGTTCCCCG TTAATGACTG CTTTTTTCAT GGTAGCTAAT TTCTTTAAGT 2580
AAAAACTTTG ATTTGAGTGA TGATGTTGTA CTGTTACACT TGCACCACAA GGGCATATAT 2640
AGAGCACAAG ACATACACAA CAACTTGCAA AACTAACTTT TGTTGGAGCA TTTCGAGGAA 2700
AATGGGGAGT AGCAGGCTAA TCTGAGGGTA ACATTAAGGT TTCATGTATT AATTTGTTGC 2760
AAACATGGAC TTAGTGTGAG GAAAAAGTAC CAAAATTTTG TCTCACCCTG ATTTCAGTTA 2820
TGGAAATTAC ATTATGAAGC TGTGCTAGAG AAGATGTTTA TTCTAGTCCA GCCACCCACC 2880
TTATGCAAGT CTGCTTTTAG CTTGATTCAA AAACTGATTT AATTTACATT GCTAAATGTG 2940
CATACTTCGA GCCTATGTCG CTTTAATTCG AGTAGGATGT ATATATTAGT ACATAAAAAA 3000
TCATGTTTGA ATCATCTTTC ATAAAGTGAC AAGTCAATTG TCCCTTCTTG TTTGGCACTA 3060
TATTCAATCT GTTAATGCAA ATTATCCAGT TATACTTAGC TAGATCCTAC GCAGCAGGTC 3120
TCATCAAGAC GATCTACCCG AGTAACAATC TCCAGGAGAT CAAATACCTT CCCAAGAAGG 3180
TTAAAGATGC AGTCAAAAGA TTCAGGACTA ATTGCATCAA GAACACAGAG AAAGACATAT 3240
TTCTCAAGAT CAGAAGTACT ATTCCAGTAT GGACGATTCA AGGCTTGCTT CATAAACCAA 3300
GGCAAGTAAT AGAGATTGGA GTCTCTAAAA AGGTAGTTCC TACTGAATCT AAGGCCATGC 3360
ATGGAGTCTA AGATTCAAAT CGAGGATCTA ACAGAACTCG CCGTGAAGAC TGGCGAACAG 3420
TTCATACAGA GTCTTTTACG ACTCAATGAC AAGAAGAAAA TCTTCGTCAA CATGGTGGAG 3480
CACGACACTC TGGTCTACTC CAAAAATGTC AAAGATACAG TCTCAGAAGA CCAAAGGGCT 3540
ATTGAGACTT TTCAACAAAG GATAATTTCG GGAAACCTCC TCGGATTCCA TTGCCCAGCT 3600

ATCTGTCACT TCATCGAAAG GACAGTAGAA AAGGAAGGTG GCTCCTACAA ATGCCATCAT 3660

TGCGATAAAG GAAAGGCTAT CATTCAAGAT GCCTCTGCCG ACAGTGGTCC CAAAGATGGA 3720

CCCCCACCCA CGAGGAGCAT CGTGGAAAAA GAAGACGTTC CAACCACGTC TTCAAAGCAA 3780

GTGGATTGAT GTGACATCTC CACTGACGTA AGGGATGACG CACAATCCCA CTATCCTTCG 3840

CAAGACCCTT CCTCTATATA AGGAAGTTCA TTTCATTTGG AGAGGACACG CTGAAATCAC 3900

CAGTCTCTCT CTATAAATCT ATCTCTCTCT CTATAACCAT GGACCCAGAA CGACGCCCGG 3960

CCGACATCCG CCGTGCCACC GAGGCGGACA TGCCGGCGGT CTGCACCATC GTCAACCACT 4020

ACATCGAGAC AAGCACGGTC AACTTCCGTA CCGAGCCGCA GGAACCGCAG GAGTGGACGG 4080

ACGACCTCGT CCGTCTGCGG GAGCGCTATC CCTGGCTCGT CGCCGAGGTG GACGGCGAGG 4140

TCGCCGGCAT CGCCTACGCG GGCCCCTGGA AGGCACGCAA CGCCTACGAC TGGACGGCCG 4200

AGTCGACCGT GTACGTCTCC CCCCGCCACC AGCGGACGGG ACTGGGCTCC ACGCTCTACA 4260

CCCACCTGCT GAAGTCCCTG GAGGCACAGG GCTTCAAGAG CGTGGTCGCT GTCATCGGGC 4320

TGCCCAACGA CCCGAGCGTG CGCATGCACG AGGCGCTCGG ATATGCCCCC CGCGGCATGC 4380

TGCGGGCGGC CGGCTTCAAG CACGGGAACT GGCATGACGT GGGTTTCTGG CAGCTGGACT 4440

TCAGCCTGCC GGTACCGCCC CGTCCGGTCC TGCCCGTCAC CGAGATCTGA TCTCACGCGT 4500

CTAGGATCCG AAGCAGATCG TTCAAACATT TGGCAATAAA GTTTCTTAAG ATTGAATCCT 4560

GTTGCCGGTC TTGCGATGAT TATCATATAA TTTCTGTTGA ATTACGTTAA GCATGTAATA 4620

ATTAACATGT AATGCATGAC GTTATTTATG AGATGGGTTT TTATGATTAG AGTCCCGCAA 4680

TTATACATTT AATACGCGAT AGAAAACAAA ATATAGCGCG CAAACTAGGA TAAATTATCG 4740

CGCGCGGTGT CATCTATGTT ACTAGATCGG GAAGATCCTC TAGAGTCGAC CTGCAGGCAT 4800

GCAAGCTT 4808

**FIGURE 1**

1. Action : Transform corn embryos (e.g. H99) with male-sterility gene S, linked to herbicide resistance gene bar (Example 5)
Result : transformed plants with genotype S/s

2. Action : Transform corn embryos (e.g. H99) with fertility-restorer gene R (Example 6)
Result : transformed plants with genotype R/r

3. Action : Transform corn embryos (e.g; H99) with maintainer gene P (Example 3)
Result : transformed plants with genotype P/p

4. Action : Cross S/s,r/r x s/s,R/r.
Select offspring for presence of both S and R genes by means of PCR.
Result : plants with genotype S/s,R/r

5. Action : Self selected plants of 4 (optional)
Result : Progeny plants with 9 different genotypes

| gamete ♀↓ ♂→ | S,R | S,r | s,R | s,r |
|---|---|---|---|---|
| S,R | S/S,R/R | S/S,R/r | S/s,R/R | S/s,R/r |
| S,r | S/S,R/r | S/S,r/r** | S/s,R/r | S/s,r/r** |
| s,R | S/s,R/R | S/s,R/r | s/s,R/R | s/s,R/r |
| s,r | S/s,R/r | S/s,r/r** | s/s,R/r | s/s,r/r |

** male-sterile plants

6. Action : self male-fertile progeny plants of 5 (Optional)
Result :

6.1. Self of S/S,R/R : 100 % male-fertile plants
Self of S/s,R/R : 100 % male-fertile plants
Self of s/s,R/R : 100 % male-fertile plants
Self of s/s,R/r : 100 % male-fertile plants
Self of s/s,r/r : 100 % male-fertile plants

6.2 Self of S/s,R/r : Same progeny as in 5
13/16 male-fertile plants with 4/13 herbicide sensitive

6.3 Self of S/S,R/r : Progeny as follows :

| gamete ♀↓ ♂→ | S,R | S,r |
|---|---|---|
| S,R | S/S,R/R | S/S,R/r |
| S,r | S/S,R/r | S/S,r/r** |

** male-sterile plants

Thus : 3/4 male-fertile plants, 0% herbicide sensitive All male-sterile plants are of genotype S/S,r/r

7. Action : Cross

♀: P/p (from 3) x ♂ : S/s,R/r (from 4)

this equals in fact

♀: s/s,r/r,P/p x ♂ : S/s,R/r,p/p

Result : Progeny with the following genotypes

| gamete ♀↓ ♂→ | S,R,p | S,r,p | s,R,p | s,r,p |
|---|---|---|---|---|
| s,r,P | S/s,R/r,P/p | S/s,r/r,P/p | s/s,R/r,P/p | s/s,r/r,P/p |
| s,r,p | S/s,R/r,p/p | S/s,r/r,p/p | s/s,R/r,p/p | s/s,r/r,p/p |

8. Action : From offspring of 7, select plants with genotype S/s,r/r,P/p by screening, by means of PCR and/or Southern blotting, for presence of S and P gene and absence of R gene.
Result : plants with genotype S/s,P/p

9. Action : Self plants with genotype S/s,P/p (from 8)
Result : progeny with the following genotypes

| gamete ♀↓ ♂→ | S,P | S,p | s,P | s,p |
|---|---|---|---|---|
| S,P | S/S,P/P | S/S,P/p | S/s,P/P | S/s,P/p |
| S,p | S/S,P/p | S/S,p/p** | S/s,P/p | S/s,p/p** |
| s,P | S/s,P/P | S/s,P/p | s/s,P/P | s/s,P/p |
| s,p | S/s,P/p | S/s,p/p** | s/s,P/p | s/s,p/p |

** male-sterile plants
Shaded genotypes cannot develop because male gametes (pollen) are killed off by expression of the maintainer gene P.

10. Action : self male fertile plants of 9.
Result

10.1. Self of s/s,P/p : 100 % male-fertile plants
Self of s/s,p/p : 100 % male-fertile plants

10.2 Self of S/s,P/p : Same progeny as in 9
5/8 male-fertile plants with
2/5 herbicide sensitive

10.3 Self of S/S,P/p : Progeny as follows :

| gamete ♀ ♂→ ↓ | S,P | S,p |
|---|---|---|
| S,P | S/S,P/P | S/S,P/p |
| S,p | S/S,P/p | S/S,p/p** |

** male-sterile plants
Shaded genotypes cannot develop because male gametes (pollen) are killed off by expression of the maintainer gene P.

FINAL RESULT

- 1/2 male-fertile plants, 0% herbicide sensitive. All these plants are maintainer plants
- 1/2 male sterile plants. All homozygous for the male-sterility gene S.

## Claims

**1.** A cell of a maintainer plant, the nuclear genome of which contains: 1) at a first locus, a male-sterility genotype in homozygous condition; and 2) at a second locus, a maintainer gene in heterozygous condition; said first and second loci preferably being unlinked; said maintainer gene being a foreign DNA sequence, preferably a foreign chimaeric DNA sequence, including:

a) a fertility-restorer gene that comprises:

i) a fertility-restorer DNA encoding a restorer RNA and/or protein or polypeptide which, when produced or overproduced in cells, preferably stamen cells, of said plant, prevents phenotypic expression of said nudear male-sterility genotype which would render said plant male-sterile in the absence of said restorer RNA, protein or polypeptide in said stamen cells and
ii) a restorer promoter capable of directing expression of said fertility-restorer DNA at least in said cells, preferably said stamen cells, so that said phenotypic expression of said nudear male-sterility genotype is prevented, said fertility-restorer DNA being in the same transcriptional unit as, and under the control of, said restorer promoter and

b) a pollen-lethality gene that is selectively expressed in microspores and/or pollen of said plant to prevent the production of functional pollen and that comprises:

iii) a pollen-lethality DNA coding for a pollen lethality RNA and/or protein or polypeptide that, when produced or overproduced in said microspores and/or pollen, significantly disrupts the metabolism, functioning and/or development of said microspores and/or pollen and
iv) a pollen-specific promoter capable of directing expression of said pollen-lethality DNA selectively in said microspores and/or pollen said pollen-lethality DNA being in the same transcriptional unit as, and under the control of, said pollen-specific promoter.

2. The cell of claim 1, in which said maintainer gene also contains, preferably in said second locus, a first marker gene which comprises:

v) a first marker DNA encoding a first marker RNA and/or protein or polypeptide which, when present at least in a first specific tissue or specific cells of said plant, renders said plant easily separable from other plants which do not contain said first marker RNA, protein or polypeptide at least in said first specific tissue or specific cells and

vi) a first marker promoter capable of directing expression of said first marker DNA at least in said first specific tissue or spedfic cells, said first marker DNA being in the same transcriptional unit as, and under the control of, said first marker promoter.

3. The cell of claim 1 or 2, in which said male-sterility genotype is foreign to said plant and is a male-sterility gene that is a foreign DNA sequence, preferably a foreign chimaeric DNA sequence, comprising:

1) a male-sterility DNA encoding a sterility RNA and/or protein or polypeptide which, when produced or overproduced in said stamen cells of said plant in the absence of said restorer RNA, protein or polypeptide, significantly disturbs the metabolism, functioning and/or development of said stamen cells and

2) a sterility promoter capable of directing expression of said male-sterility DNA selectively in said stamen cells, said male-sterility DNA being in the same transcriptional unit as, and under the control of, said sterility promoter.

4. The cell of claim 3 in which said male-sterility genotype also contains, preferably in said first locus, a second marker gene which comprises:

3) a second marker DNA encoding a second marker RNA and/or protein or polypeptide which, when present at least in a second specific tissue or specific cells of said plant, renders said plant easily separable from other plants which do not contain said second marker RNA, protein or polypeptide at least in said second specific tissue or specific cells and

4) a second marker promoter capable of directing expression of said second marker DNA at least in said specific tissue or specific cells, said second marker DNA being in the same transcriptional unit as, and under the control of, said second marker promoter.

5. The cell of claim 3 or 4, in which said male sterility DNA encodes Bamase.

6. The cell of any one of claims 3 to 5, in which said sterility promoter is selected among the TA29 promoter of Nicotiana tabacum and the promoters of SEQ ID nos. 7 to 10.

7. The cell of claim 5 or 6, in which said restorer DNA encodes Barstar.

8. The cell of any one of claims 1 to 7, in which said restorer promoter is selected among the TA29 promoter of Nicotiana tabacum and the promoters of SEQ ID nos. 7 to 10.

9. The cell of claim 1 or 2, in which said male-sterility genotype is endogenous to said plant and is homozygous for a recessive allele.

10. The cell of claim 9, in which said fertility-restorer gene is the dominant allele of the endogenous male-sterility genotype, preferably under the control of its natural promoter.

11. The cell of any one of claims 1 to 10, in which said pollen-lethality DNA encodes a ribonuclease, preferably RNAseT1 or Bamase.

12. The cell of any one of claims 1 to 11, in which said pollen-specific promoter is the Zm13 promoter of SEQ ID no. 1.

13. The cell of any one of claims 2 to 12, in which said first marker DNA or said second marker DNA is: an herbicide resistance gene, particularly an sfr or sfrv gene; a gene encoding a modified target enzyme for an herbicide having lower affinity for the herbicide, particularly a modified 5-enolpyruvylshikimate-3 phosphate synthase as a target for glyphosate or a modified glutamine synthetase as a target for a glutamine synthetase inhibitor such as phosphinotricine; a gene encoding a protein which confers resistance to the herbicide sulfonylurea; a gene encoding a protein which confers resistance to the herbicide, bromoxynil; a gene encoding a protein which confers resistance to the herbicide 2,4 D; a gene encoding a protein or a polypeptide conferring a color to at least said first or second specific tissue or specific cells, particularly the A1 gene or the glucuronidase gene; a gene encoding a protein or a polypeptide conferring a stress tolerance to said plant, particularly a gene encoding Mn-superoxide dismutase; a gene encoding a protein or a polypeptide conferring a disease or pest resistance, particularly a gene encoding a Bacillus thuringiensis endotoxin that confers insect resistance; or a gene encoding a bactericidal peptide that confers a baderial resistance.

**14.** The cell of any one of claims 2 to 13, in which said first marker promoter or said second marker promoter is: a constitutive promoter, particularly a 35S promoter, a 35S'3 promoter, a PNOS promoter or a POCS promoter, a wound-inducible promoter, particularly a TR1' or TR2' promoter; a promoter which directs gene expression selectively in plant tissue having photosynthetic activity, particularty an SSU promoter; or a promoter which directs gene expression selectively in leaf cells, petal cells or seed cells, particularly seed coat cells.

**15.** The cell of any one of claims 2 to 12, wherein said first marker DNA and said second marker DNA are different.

**16.** A plant cell culture consisting essentially of the plant cells of any one of claims 1 to 15.

**17.** A plant, particularty corn, oilseed rape, wheat, rice, sunflower, sugar beet, tomato, lettuce, peppers, sorghum, soybean, pea, alfalfa, grasses, clovers, carrot, cabbages, leek, onion, tobacco, petunia, cacao and citrus, more particularly corn, oilseed rape, wheat and rice, consisting essentially of the plant cells of any one of claims 1 to 15.

**18.** A seed of the plant of claim 17, consisting essentially of the plant cells of any one of claims 1 to 15.

**19.** A maintainer gene for maintaining a homogenous population of male-sterile plants, comprising:

a) a fertility-restorer gene that comprises:

i) a fertility-restorer DNA encoding a restorer RNA and/or protein or polypeptide which, when produced or overproduced in cells, preferably stamen cells, of a plant, prevents phenotypic expression of a nuclear male-sterility genotype which would render said plant male sterile in the absence of said restorer RNA, protein or polypeptide in said stamen cells and
ii) a restorer promoter capable of directing expression of said fertility-restorer DNA at least in said cells, preferably said stamen cells, so that said phenotypic expression of said nudear male-sterility genotype is prevented, said fertility-restorer DNA being in the same transcriptional unit as, and under the control of, said restorer promoter, and

b) a pollen-lethality gene that is selectively expressed in microspores and/or pollen of said plant to prevent the production of functional pollen and that comprises:

iii) a pollen-lethality DNA coding for a pollen lethality RNA and/or protein or polypeptide that, when produced or overproduced in said microspores and/or pollen, significantly disrupts the metabolism, functioning and/or development of said microspores and/or pollen and
iv) a pollen-specific promoter capable of directing expression of said pollen-lethality DNA selectively in said microspores and/or pollen said pollen-lethality DNA being in the same transcriptional unit as, and under the control of, said pollen-specific promoter.

**20.** A vector for transforming a cell of a plant, comprising the maintainer gene of claim 19.

**21.** A method to maintain a homogeneous population of male-sterile plants or their seed, the nuclear genome of which contain, at said first locus of any one of claims 1, 3-6 and 9, said male-sterility genotype of any one of claims 1, 3-6 and 9 in homozygous condition; said method comprising the step of crossing said male-sterile plants with the plant of claim 17.

**Patentansprüche**

1. Die Zelle einer Maintainerpflanze, deren nukleäres Genom folgendes enthält:

   1) an einem ersten Locus, einen männlich-sterilen Genotyp in homozygotem Zustand; und
   2) an einem zweiten Locus ein Maintainergen in heterozygotem Zustand;

   wobei die besagten Loci vorzugsweise unverknüpft sind; und
   es sich bei dem besagten Maintainergen um eine fremde DNA-Sequenz, vorzugsweise eine fremde chimärische DNA-Sequenz, handelt, einschließlich:

   a) ein Fertilitätsrestorergen, welches folgendes enthält:

      i) eine Fertilitätsrestorer-DNA, welche ein/e Restorer-RNA und/oder - Protein oder Polypeptid codiert, die/das, wenn sie/es in den Zellen, vorzugsweise den Staubblattzellen, der besagten Pflanze produziert oder überproduziert wird, die phänotypische Expression des besagten nukleären männlich-sterilen Genotyps verhindert, der die besagte Pflanze in Abwesenheit der/des besagten Restorer-RNA, - Proteins oder Polypeptids männlich steril machen würde; und
      ii) einen Restorer-Promotor, welcher geeignet ist, die Expression der besagten Fertilitätsrestorer-DNA in zumindest den besagten Zellen, vorzugsweise in den besagten Staubblattzellen, zu steuern, so dass die phänotypische Expression des besagten nukleären männlich-sterilen Genotyps verhindert wird, wobei sich die besagte Fertilitätsrestorer-DNA in der gleichen Transkriptionseinheit wie und unter der Kontrolle des besagten Restorer-Promotors befindet; und

   b) ein Pollenletalitätsgen, das in den Mikrosporen und/oder Pollen der besagten Pflanze selektiv exprimiert wird, um die Produktion von bestäubungsfähigem Pollen zu verhindern und welches folgendes beinhaltet:

      iii) eine Pollenletalitäts-DNA, die für ein/e pollenletale/s RNA und/oder Protein oder Polypeptid codiert, welche/s, wenn sie/es in den besagten Mikrosporen und/oder Pollen produziert oder überproduziert wird, deren Stoffwechsel, Funktionsfähigkeit und/oder Entwicklung erheblich beeinträchtigt und
      iv) Einen pollenspezifischen Promotor, der geeignet ist, die Expression der besagten Pollenletalitäts-DNA selektiv in den besagten Mikrosporen und/oder Pollen zu steuern, wobei sich die besagte Pollenletalitäts-DNA in der gleichen Transkriptionseinheit wie und unter Kontrolle des besagten pollenspezifischen Promotors befindet.

2. Die Zelle von Anspruch 1, in der das besagte Maintainergen, vorzugsweise am zweiten Locus, auch ein erstes Markierungsgen enthält, welches folgendes beinhaltet:

   v) eine erste Markierungs-DNA, die eine erste Markierungs-RNA, ein erstes Markierungs-Protein oder -Polypeptid codiert, welches, wenn es zumindest in einem ersten spezifischen Gewebe oder spezifischen Zellen der besagten Pflanze enthalten ist, die besagte Pflanze leicht von anderen Pflanzen unterscheidbar macht, die nicht die besagte erste Markierungs-RNA, das erste Markierungs-Protein oder -Polypeptid zumindest in dem besagten ersten spezifischen Gewebe oder spezifischen Zellen enthalten und,
   vi) einen ersten Markierungs-Promotor, der geeignet ist, die Expression der besagten ersten Markierungs-DNA zumindest in dem besagten spezifischen Gewebe oder spezifischen Zellen zu steuern, wobei sich die besagte erste Markierungs-DNA in der gleichen Transkriptionseinheit wie und unter der Kontrolle des besagten ersten Markierungs-Promotors befindet.

3. Die Zelle von Anspruch 1 oder 2, wobei der besagte männlich-sterile Genotyp der besagten Pflanze fremd ist und es sich um ein Männliches-Sterilitäts-Gen handelt, das aus einer fremden DNA-Sequenz besteht, vorzugsweise aus einer fremden chimärischen DNA-Sequenz, die folgendes enthält:

   1) eine Männliche-Sterilitäts-DNA, welche ein/e Sterilitäts-RNA, und/oder-Protein oder Polypeptid codiert, die/das, wenn sie/es in den Zellen, vorzugsweise den Staubblattzellen, der besagten Pflanze in Abwesenheit des/der besagten Restorer-DNA, Proteins oder Polypeptids produziert oder überproduziert wird, Stoffwechsel, Funktionsfähigkeit und/oder Entwicklung der besagten Staubblattzellen erheblich beeinträchtigt und
   2) einen Sterilitätspromotor, der geeignet ist, die Expression der besagten Männlichen-Sterilitäts-DNA selektiv in den besagten Staubblattzellen zu steuern, wobei sich die besagte Männliche-Sterilitäts-DNA in der gleichen Transkriptionseinheit wie und unter der Kontrolle des besagten Sterilitätspromotors befindet.

**4.** Die Zelle von Anspruch 3, in der der besagte männlich-sterile Genotyp, vorzugsweise am ersten Locus, auch ein zweites Markierungsgen enthält, welches folgendes beinhaltet:

3) eine zweite Markierungs-DNA, welche ein/e zweite/s Markierungs-RNA, - und/oder Protein oder Polypeptid codiert, durch welche/s die besagte Pflanze, wenn sie/es in mindestens einem zweiten spezifischen Gewebe oder in spezifischen Zellen vorkommt, leicht von anderen Pflanzen zu unterscheiden ist, die nicht die/das besagte zweite Markierungs-RNA, - Protein oder Polypeptid in mindestens dem besagten zweiten spezifischen Gewebe oder in spezifischen Zellen enthalten und
4) einen zweiten Markierung-Promotor, der geeignet ist, die Expression der besagten zweiten Markierungs-DNA zumindest in das besagte spezifische Gewebe oder spezifische Zellen zu leiten, wobei sich die zweite Markierungs-DNA in der gleichen Transkriptionseinheit und unter der Kontrolle des zweiten Markierungs-Promotor befindet.

**5.** Die Zelle von Anspruch 3 oder 4, wobei die besagte Männliche-Sterilitäts-DNA Barnase codiert.

**6.** Die Zelle eines der Ansprüche 3 bis 5, wobei der besagte Sterilitätspromotor aus dem TA29-Promotor von Nicotiana Tabacum oder den Promotoren mit den SEQ ID Nummern 7 bis 10 ausgewählt ist.

**7.** Die Zelle von Anspruch 5 oder 6, wobei die besagte Restorer-DNA Barstar codiert.

**8.** Die Zelle von einem der Ansprüche 1 bis 7, wobei der besagte Restorerpromotor aus dem TA29-Promotor von Nicotiana Tabacum oder den Promotoren mit den SEQ ID Nummern 7 bis 10 ausgewählt ist.

**9.** Die Zelle von Anspruch 1 oder 2, wobei der besagte Männliche-Sterilitäts-Genotyp endogen von der besagten Pflanze produziert wird und für ein rezessives Allel homozygot ist.

**10.** Die Zelle von Anspruch 9, in der das besagte Fertilitätsrestorergen das dominante Allel des endogenen männlichsterilen Genotyps ist, vorzugsweise unter der Kontrolle seines natürlichen Promoters.

**11.** Die Zelle eines der Ansprüche 1 bis 10, in der die besagte Pollenletalitäts-DNA eine Ribonuklease codiert, vorzugsweise RNAseT1 oder Barnase.

**12.** Die Zelle eines der Ansprüche von 1 bis 11, in der es sich bei dem besagten pollenspezifischen Promotor um den Zm13-Promotor mit der SEQ ID Nummer 1 handelt.

**13.** Die Zelle eines der Ansprüche von 2 bis 12, in der die besagte erste oder die besagte zweite Markierungs-DNA einer der folgenden Beschreibungen entspricht: ein Herbizid-Resistenzgen, insbesondere ein sfr oder sfrv -Gen; ein Gen, das ein modifiziertes Zielenzym für ein Herbizid mit verringerter Affinität für das Herbizid codiert, insbesondere eine modifizierte 5-Enolpyruvylshikimate-3-Phosphat-Synthase als Zielenzym für Glyphosat oder eine modifizierte Glutamin-Synthase als Zielenzym für einen Glutamin-Synthase-Inhibitor wie Phosphinotrizin; ein Gen, das ein Protein codiert, welches Resistenz gegen Sulfonylharnstoffherbizide bewirkt; ein Gen, das ein Protein codiert, welches Resistenz gegen das Herbizid Bromoxynil bewirkt; ein Gen, das ein Protein codiert, welches Resistenz gegen das Herbizid 2,4 D bewirkt; ein Gen, das ein Protein oder Polypeptid codiert, welches zumindest das besagte erste oder zweite spezifische Gewebe oder spezifische Zellen einfärbt, insbesondere das A1-Gen oder das Glucuronidase-Gen; ein Gen, das ein Protein oder Polypeptid codiert, welches der besagten Pflanze eine Belastungstoleranz verleiht, insbesondere ein Gen, das Mn-Superoxid-Dismutase codiert; ein Gen, das ein Protein oder Polypeptid codiert, welches eine Resistenz gegen Krankheits- oder Schädlingsbefall bewirkt, insbesondere ein Gen, das ein Endotoxin des Bacillus Thuringensis codiert, welches Resistenz gegen Insekten bewirkt; oder ein Gen, das ein bakterizides Peptid codiert, welches bakterielle Resistenz verleiht.

**14.** Die Zelle eines der Ansprüche 2 bis 13, in der der besagte erste oder der besagte zweite Markierungs-Promotor einer der folgenden Beschreibungen entspricht: ein konstitutiver Promotor, insbesondere ein 35S-Promotor, ein 35S'3-Promotor, ein PNOS-Promotor oder ein POCS-Promotor; ein wundinduzierbarer Promotor, insbesondere ein TR1'- oder TR2'-Promotor; ein Promotor, der die Genexpression selektiv in Pflanzengewebe mit Photosyntheseaktivität lenkt, insbesondere ein SSU-Promotor, der die Genexpression selektiv in Blattzellen, Blütenblattzellen oder Samenzellen, insbesondere Samenhüllenzellen lenkt.

**15.** Die Zelle eines der Ansprüche 2 bis 12, in der sich die besagten ersten und zweiten Markierungs-DNAs unterscheiden.

**16.** Eine Pflanzenzellenkultur, die im Wesentlichen aus Zellen eines der Ansprüche 1 bis 15 besteht.

**17.** Eine Pflanze, insbesondere Mais, Ölraps, Weizen, Reis, Sonnenblume, Zuckerrübe, Tomate, Kopfsalat, Paprikasorten, Hirse, Soja, Erbse, Alfalfa, Gräser, Kleesorten, Möhre, Kohlsorten, Lauch, Zwiebel, Tabak, Petunie, Kakao und Zitrus, vor allem Mais, Ölraps, Weizen und Reis, die im Wesentlichen aus den Zellen eines der Ansprüche 1 bis 15 bestehen.

**18.** Ein Samen der Pflanze von Anspruch 17, der im Wesentlichen aus Zellen eines der Ansprüche 1 bis 15 besteht.

**19.** Ein Maintainergen zum Erhalt einer homogenen Population männlich-steriler Pflanzen, welches folgendes enthält:

a) ein Fertilitätsrestorergen, welches folgendes enthält:

i) eine Fertilitätsrestorer-DNA, welche ein/e Restorer-RNA, und/oder - Protein oder Polypeptid codiert, die/das, wenn sie/es in den Zellen, vorzugsweise den Staubblattzellen, einer Pflanze produziert oder überproduziert wird, die phänotypische Expression eines nukleären männlich-sterilen Genotyps verhindert, der die besagte Pflanze in Abwesenheit der/des besagten Restorer-RNA, -Proteins oder Polypeptids in den besagten Staubblattzellen männlich steril machen würde, und
ii) einen Restorer-Promotor, welcher geeignet ist, die Expression der besagten Fertilitätsrestorer-DNA in zumindest den besagten Zellen, vorzugsweise in den besagten Staubblattzellen, zu steuern, so dass die besagte phänotypische Expression des besagten nukleären männlich-sterilen Genotyps verhindert wird, wobei sich die besagte Fertilitätsrestorer-DNA in der gleichen Transkriptionseinheit wie und unter der Kontrolle des besagten Restorer-Promotors befindet; und

b) ein Pollenletalitätsgen, das selektiv in den Mikrosporen und/oder Pollen der besagten Pflanze exprimiert wird, um die Produktion von bestäubungsfähigem Pollen zu verhindern und welches folgendes beinhaltet:

iii) eine Pollenletalitäts-DNA, die für ein/e pollenletale/s RNA und/oder Protein oder Polypeptid codiert, welche/s, wenn sie/es in den besagten Mikrosporen und/oder Pollen produziert oder überproduziert wird, deren Stoffwechsel, Funktionsfähigkeit und/oder Entwicklung erheblich beeinträchtigt und
iv) Einen pollenspezifischen Promotor, der geeignet ist, die Expression der besagten Pollenletalitäts-DNA selektiv in den besagten Mikrosporen und/oder Pollen zu steuern, wobei sich die besagte Pollenletalitäts-DNA in der gleichen Transkriptionseinheit wie und unter Kontrolle des besagten pollenspezifischen Promotors befindet.

**20.** Ein Vektor für die Veränderung der Zelle einer Pflanze, der das Maintainergen von Anspruch 19 enthält.

**21.** Eine Methode für den Erhalt einer homogenen Population männlich-steriler Pflanzen oder deren Saatgut, deren nukleäres Genom am besagten ersten Locus von einem der Ansprüche 1, 3-6 und 9, den besagten männlich-sterilen Genotyp eines der Ansprüche 1, 3-6 und 9 in homozygotem Zustand enthält; die besagte Methode beinhaltet den Schritt, die besagten männlich-sterilen Pflanzen mit der Pflanze von Anspruch 17 zu kreuzen.

## Revendications

**1.** Une cellule d'une plante support dont le génome nucléaire contient:

1) au premier locus, un génotype de stérilité mâle à l'état homozygote, et
2) au second locus, un gène support à l'état hétérozygote;

lesdits premier et second loci étant de préférence détachés;
ledit gène support étant une séquence ADN étrangère,
une séquence ADN étrangère chimérique de préférence, comprenant:

a) un gène restaurateur de fertilité qui comprend:

i) un ADN restaurateur de fertilité encodant un ARN et/ou une protéine ou un polypéptide restaurateur qui, lorsqu'il est produit ou produit en excès dans des cellules, de préférence des cellules étamines de ladite plante, empêche l'expression phénotypique de ce génotype nucléaire de stérilité mâle qui pourrait rendre ladite plante mâle stérile en l'absence de l'ARN, de la protéine ou du polypeptide restaurateur dans les cellules étamines et
ii) un promoteur de restauration capable de diriger l'expression de l'ADN restaurateur de fertilité au moins dans les cellules de la plante, dans les cellules étamines de préférence, afin que l'expression phénotypique dudit génotype nucléaire de stérilité mâle soit bloquée, l'ADN restaurateur de fertilité étant dans la même unité de transcription que le promoteur de restauration, et sous le contrôle de ce dernier et

b) un gène « tueur » de pollen qui s'exprime de manière sélective dans les microspores et/ou le pollen de ladite plante afin de prévenir la production du pollen fonctionnel et qui comprend :

iii) un ADN « tueur » de pollen qui code un ARN et/ou une protéine ou un polypeptide « tueur » de pollen qui, lorsqu'ils sont produits ou produits en excès dans les microspores et/ou pollen, perturbent de manière significative le métabolisme, le fonctionnement et/ou le développement de ces micropores et/ou pollen et
iv) un promoteur spécifique au pollen capable de diriger l'expression de l'ADN « tueur » de pollen de manière sélective dans les microspores et/ou pollen, l'ADN « tueur » de pollen étant dans la même unité de transcription que le promoteur spécifique au pollen et sous le contrôle de ce dernier.

**2.** La cellule de la revendication 1, dans laquelle le gène support contient également, dans ledit second locus de préférence, un premier gène marqueur qui comprend:

v) Un premier ADN marqueur codant un premier ARN et/ou une protéine ou un polypéptide marqueur qui, lorsqu'il est présent au moins dans le premier tissu spécifique ou dans les cellules spécifiques de la plante, rend cette plante facilement séparable des autres plantes qui ne contiennent pas ledit premier ARN, protéine ou polypeptide marqueur au moins dans le premier tissu spécifique ou dans les cellules spécifiques et
vi) Un premier promoteur marqueur capable de diriger l'expression du premier ADN marqueur au moins dans le premier tissu spécifique ou dans les cellules spécifiques, le premier ADN marqueur étant dans la même unité de transcription que le premier promoteur marqueur, et sous le contrôle de ce dernier.

**3.** La cellule des revendications 1 ou 2, dans laquelle le génotype de stérilité mâle est étranger à ladite plante et est un gène de stérilité mâle c'est-à-dire une séquence ADN étrangère, une séquence ADN étrangère chimérique de préférence, comprenant:

1) un ADN de stérilité mâle codant un ARN et/ou une protéine ou un polypeptide stérile qui, lorsqu'il est produit ou produit en excès dans les cellules étamines de ladite plante en l'absence de l'ARN, de la protéine ou du polypeptide restaurateur, perturbe de manière significative le métabolisme, le fonctionnement et/ou le développement des cellules étamines et
2) un promoteur de stérilité capable de diriger l'expression de l'ADN de stérilité mâle de manière sélective dans lesdites cellules étamines, l'ADN de stérilité mâle étant dans le même unité de transcription que le promoteur de stérilité, et sous le contrôle de ce dernier.

**4.** La cellule de la revendication 3 dans laquelle le génotype de stérilité mâle contient également, de préférence dans le premier locus, un second gène marqueur qui est constitué d' :

3) un second ADN marqueur codant un second ARN et/ou protéine ou polypeptide marqueur qui, lorsqu'il est présent au moins dans un second tissu spécifique ou dans des cellules spécifiques de la plante rend cette plante facilement séparable des autres plantes qui ne contiennent pas ledit second ARN, protéine ou polypeptide marqueur au moins dans le second tissu spécifique ou dans les cellules spécifiques et
4) un second promoteur marqueur capable de diriger l'expression du second ADN marqueur au moins dans le tissu spécifique ou les cellules spécifiques, le second ADN marqueur étant dans la même unité de transcription que le second promoteur marqueur, et sous le contrôle de ce dernier.

**5.** La cellule des revendications 3 ou 4, dans laquelle l'ADN de stérilité mâle encode le barnase.

**6.** La cellule de l'une des quelconques revendications 3 à 5, dans laquelle le promoteur de stérilité est sélectionné parmi le promoteur TA29 de nicotiana tabacum et les promoteurs SEQ ID numérotés de 7 à 10.

**7.** La cellule des revendications 5 ou 6, dans laquelle l'ADN restaurateur encode le barstar.

**8.** La cellule de l'une des quelconques revendications 1 à 7, dans laquelle le promoteur restaurateur est sélectionné parmi le promoteur TA29 de nicotiana tabacum et les promoteurs SEQ ID numérotés de 7 à 10.

**9.** La cellule des revendications 1 ou 2, dans laquelle le génotype de stérilité mâle est endogène à cette plante et est homozygote pour un allèle récessif.

**10.** La cellule de la revendication 9, dans laquelle le gène restaurateur de fertilité est l'allèle dominant du génotype endogène de stérilité mâle, sous le contrôle du promoteur naturel de préférence.

**11.** La cellule de l'une des quelconques revendications de 1 à 10, dans laquelle l'ADN « tueur » de pollen encode une ribonucléase, de type RNAseT1 ou Barnase de préférence.

**12.** La cellule de l'une des quelconques revendications 1 à 11, dans laquelle le promoteur spécifique au pollen est le promoteur Zm13 de SEQ ID n°1.

**13.** La cellule de l'une des quelconques revendications 2 à 12, dans laquelle le premier ou le second ADN marqueur est un gène resistant à l'herbicide, particulièrement s'il s'agit d'un gène sfr ou sfrv, un gène encodant une enzyme cible modifiée pour un herbicide ayant une affinité inférieure envers l'herbicide, particulièrement une 5-enolpyruvylshikimate-3-phosphate synthétase modifiée comme cible du glyphosate ou un glutamine synthétase modifié comme cible pour l'inhibiteur de glutamine synthétase tel que la phosphinotricine; un gène encodant une protéine qui lui permet de résister à l'herbicide sylfonylurée, un gène encodant une protéine qui lui permet de résister à l'herbicide bromoxynil; un gène encodant une protéine qui lui permet de résister à l'herbicide 2,4 D; un gène encodant une protéine ou un polypeptide conférant une couleur au moins au premier ou au second tissu spécifique ou aux cellules spécifiques, particulièrement le gène A1 et le gène de glucuronidase, un gène encodant une protéine ou un polypéptide conférant une tolérance au stress à ladite plante, particulièrement un gène encodant le dismutase Mn-superoxide; un gène encodant une protéine ou un polypéptide lui conférant une résistance aux maladies et aux organismes nuisibles, particulièrement le gène encodant une endotoxine Bacillus thuringiensis qui lui confère une résistance aux insectes ou un gène encodant un peptide bactericide qui lui confère une résistance aux bactéries.

**14.** La cellule de l'une des quelconques revendications 2 à 13, dans laquelle le premier ou second promoteur marqueur est un promoteur constitutif, particulièrement les promoteurs 35S, 35S'3, PNOS ou POCS; un promoteur inductible par blessure, particulièrement le promoteur TR1' ou TR2'; un promoteur qui dirige l'expression génétique de manière sélective dans le tissu de la plante ayant une activité photsynthétique, particulièrement le promoteur SSU ou un promoteur qui dirige l'expression génétique de manière sélective dans les cellules de la feuille, dans celles des pétales ou dans celles des graines, particulièrement les cellules de l'épisperme.

**15.** La cellule de l'une des quelconques revendications 2 à 12, dans laquelle le premier et le second ADN marqueur sont différents.

**16.** Une culture de cellules végétales consistant essentiellement des cellules des plantes de l'une des quelconques revendications 1 à 15.

**17.** Une plante, particulièrement le maïs, le colza, le blé, le riz, le tournesol, la bettrave sucrière, la tomate, la lettue, les poivrons, le sorgho, le soja, le pois, la luzerne, des graminées, les trèfles, la carotte, le chou, le poireau, l'onion, le tabac, le pétunia, le cacao et les agrumes, et plus particulièrement le maïs, le colza, le blé et le riz, consistant essentiellement l'une des quelconques revendications 1 à 15.

**18.** Une graine de la plante de la revendication 17, qui se constitue essentiellement des cellules de la plante de l'une des quelconques revendications 1 à 15.

**19.** Un gène support afin de maintenir une population homogène de plantes stériles mâles, comprenant:

a) un gène restaurateur de fertilité qui se compose de:

i) un ADN restaurateur de fertilité encodant un ARN et/ou une proteine ou un polypeptide restaurateur qui, lorsqu'il est produit ou produit en excès dans les cellules, estamines de préférence, de la plante, empêche l'expression phénotypique d'un génotype nucléaire de stérilité mâle qui pourrait rendre cette plante mâle stérile en l'absence de l'ARN, de la proteine ou du polypeptide restaurateur dans les cellules étamines et

ii) un promoteur de restauration capable de diriger l'expression de l'ADN restaurateur de fertilité au moins dans les cellules, étamines de préference, afin que l'expression phénotypique du génotype nucléaire de stérilité mâle soit bloquée, l'ADN restaurateur de fertilité étant dans la même unité de transcription que le promoteur de restauration, et sous le contrôle de ce dernier, et

b) un gène « tueur » de pollen qui s'exprime de manière sélective dans les microspores et/ou le pollen de ladite plante afin de prévenir la production de pollen fonctionnel, et qui comprend:

iii) un ADN « tueur » de pollen codant un ARN et/ou une proteine ou un polypeptide « tueur » de pollen qui, lorsqu'ils sont produits ou produits en excès dans lesdites microspores et/ou le pollen, perturbe de manière significative le métabolisme, le fonctionnement et/ou le développement des microspores et/ou pollen et

iv) un promoteur spécifique au pollen capable de diriger l'expression de l'ADN « tueur » de pollen de manière sélective dans les microspores et/ou pollen, l'ADN « tueur » de pollen étant dans la même unité de transcription que le promoteur specifique au pollen, et sous le contrôle de ce dernier.

**20.** Un vecteur pour transformer la cellule d'une plante, comprenant le gène de la revendication 19.

**21.** Une méthode visant à maintenir une population homogène de plantes mâles stériles ou de leur graine, dont le génome nucléaire contient au premier locus de l'une des quelconques revendications 1, 3-6 et 9, le génotype de stérilité mâle de l'une des quelconques revendications 1, 3-6 à 9 à l'état homozygote; ladite méthode comprenant l'étape qui consiste à croiser les plantes mâles stériles avec la plante de la revendication 17.